(19) Europäisches Patentamt
European Patent Office
Office européen des brevets

(11) Veröffentlichungsnummer: **0 306 505 B1**

# (12) EUROPÄISCHE PATENTSCHRIFT

(45) Veröffentlichungstag der Patentschrift: **24.03.93**

(51) Int. Cl.[5]: **C07C 255/49**, C07C 25/24, C07C 43/215, C07C 13/28, C07C 23/18, C07C 43/225, C07D 213/16, C07D 213/30, C07D 213/65, C07D 239/26, C07D 239/34

(21) Anmeldenummer: **88902103.6**

(22) Anmeldetag: **10.03.88**

(86) Internationale Anmeldenummer: **PCT/DE88/00136**

(87) Internationale Veröffentlichungsnummer: **WO 88/07523 (06.10.88 88/22)**

Die Akte enthält technische Angaben, die nach dem Eingang der Anmeldung eingereicht wurden und die nicht in dieser Patentschrift enthalten sind.

## (54) ETHINDERIVATE ALS KOMPONENTE FLÜSSIGKRISTALLINER PHASEN.

(30) Priorität: **27.03.87 DE 3710069**

(43) Veröffentlichungstag der Anmeldung: **15.03.89 Patentblatt 89/11**

(45) Bekanntmachung des Hinweises auf die Patenterteilung: **24.03.93 Patentblatt 93/12**

(84) Benannte Vertragsstaaten: **CH DE FR GB IT LI NL**

(56) Entgegenhaltungen:
**EP-A- 0 111 695**
**EP-A- 0 224 725**
**WO-A-88/02130**
**GB-A- 2 155 465**

(73) Patentinhaber: **MERCK PATENT GESELLSCHAFT MIT BESCHRÄNKTER HAFTUNG**
**Frankfurter Strasse 250 Postfach 4119**
**W-6100 Darmstadt(DE)**

(72) Erfinder: **REIFFENRATH, Volker**
**Jahnstra e 18**
**W-6101 Ro dorf(DE)**
Erfinder: **POETSCH, Eike**
**Am Buchwald 4**
**W-6109 Mühltal(DE)**
Erfinder: **KRAUSE, Joachim**
**Samuel-Morse-Str. 14**
**W-6110 Dieburg(DE)**
Erfinder: **WEBER, Georg**
**Wilhelm-Leuschner-Str. 38**
**W-6106 Erzhausen(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services
(3.10/3.5x/3.0.1)

CHEMICAL ABSTRACTS, Band 94, Nr. 17, 27. April 1981, Columbus, Ohio, US; K. PRAEFCKE et al: "Liquid crystal compounds. VIII. 1,2-Bis(trans-4-alkylcyclohexyl) ethanes, -ethenes and -ethynes - new liquid crystal compounds", siehe S. 714, Zusammenfassung Nr. 139275c

CHEMICAL ABSTRACTS, Band 107, Nr. 16, 19. Oktober 1987, Columbus, Ohio, US; Zusammenfassung Nr. 145003r, siehe S. 709 & JP-A-61280441

MOLECULAR CRYSTALS AND LIOUID CRYSTALS, Band 111, Nr. 3 und 4, 1984,Gordon and Breach Science Publishers, US; M. PETRZILKA: "Apolar acetylenic liquid crystals", S. 347-358, siehe S. 354, Tabelle und S. 355, Spalte 4

MOLECULAR CRYSTALS AND LIOUID CRYSTALS, Band 111, Nr. 3 und 4, 1984, Gordon and Breach Science Publishers, US; M. PETRZILKA: "Polar acetylenic liquid crystals with broad mesomorphic ranges. The positional influence of different C,C-elements on the transition temperatures", S. 329-346, siehe S. 335, Tabelle

## Beschreibung

Die Erfindung betrifft Ethinderivate der Formel I

$R^1$-$(A^1$-$Z^1)_m$-$A^3$-C≡C-$A^4$-$(Z^2$-$A^2)_n$-$R^2$ I

worin

$R^1$ und $R^2$ jeweils unabhängig voneinander einen unsubstituierten, einen einfach durch -CN oder einen mindestens einfach durch Halogen substituierten Alkyl- oder Alkenylrest mit 1 bis 15 C-Atomen, wobei in diesen Resten auch eine oder mehrere $CH_2$-Gruppen jeweils unabhängig voneinander durch -O-, -S-, -CO-, -O-CO-, -O-COO-, -CO-O-oder -C≡C- so ersetzt sein können, daß Heteroatome nicht direkt miteinander verknüpft sind, einer der Reste $R^1$ und $R^2$ auch H, Halogen, -CN oder -NCS,

$A^1$ und $A^2$ jeweils unabhängig voneinander einen
a) 1,4-Phenylenrest,
worin auch eine oder mehrere CH-Gruppen durch N ersetzt sein können,
b) trans-1,4-Cyclohexylenrest,
worin auch eine oder zwei nicht benachbarte $CH_2$-Gruppen durch -O- und/oder -S- ersetzt sein können,
wobei die Reste a) und b) ein- oder mehrfach durch Halogen, Cyano und/oder $CH_3$ substituiert sein können,

$Z^1$ und $Z^2$ jeweils unabhängig voneinander -CO-O-, -O-CO-, -$CH_2$O-, -O$CH_2$-, -$CH_2CH_2$-, -C≡C- oder eine Einfachbindung,

m und n jeweils unabhängig voneinander 0 oder 1,
und

$A^3$ und $A^4$ jeweils unabhängig voneinander einen
a) 1,4-Phenylen-, Pyridin-2,5-diyl- oder Pyrimidin-2,5-diyl-Rest,
b) trans-1,4-Cyclohexylenrest,
wobei die Reste a) und b) ein- oder mehrfach durch Halogen, Cyano und/oder $CH_3$ substituiert sein können,

bedeutet, mit den Maßgaben daß,
a) eine und nur eine der Gruppen $A^3$ oder $A^4$ einen Pyridin-, Pyrimidin- oder Cyclohexylenrest bedeutet,
b) $R^2$ Halogen, einen mindestens einfach durch Halogen substituierten Alkenylrest mit 1 bis 15 C-Atomen oder einen Perfluoralkylrest mit 1 bis 15 C-Atomen bedeutet, falls m = n = 0, $A^3$ trans-1,4-Cyclohexylen und $A^4$ unsubstituiertes 1,4-Phenylen bedeutet,
bedeutet.

Der Einfachheit halber bedeuten im folgenden Phe eine unsubstituierte 1,4-Phenylengruppe, PheX eine substituierte 1,4-Phenylengruppe (wobei X Halogen, CN und/oder $CH_3$ bedeutet), Cyc eine 1,4-Cyclohexylengruppe, Che eine 1,4-Cyclohexenylengruppe, Cha eine 1,4-Cyclohexadienylengruppe, Dio eine 1,3-Dioxan-2,5-diylgruppe, Dit eine 1,3-Dithian-2,5-diylgruppe, Pyd eine Pyridin-2,5-diylgruppe, Pyr eine Pyrimidin-2,5-diylgruppe, Pyz eine Pyrazin-2,5-diylgruppe, Pyn eine Pyridazin-3,6-diylgruppe, Bco eine 1,4-Bicyclo(2.2.2)octylengruppe und Biphe eine 4,4'-Biphenylylgruppe, worin auch eine oder mehrere CH-Gruppen durch N ersetzt sein können.

Die Verbindungen der Formel I können als Komponenten flüssigkristalliner Phasen verwendet werden, insbesondere für Displays, die auf dem Prinzip der verdrillten Zelle, dem Guest-Host-Effekt, dem Effekt der Deformation aufgerichteter Phasen oder dem Effekt der dynamischen Streuung beruhen.

Verbindungen der Formel I sind vorzugsweise auch geeignet für die Verwendung als Komponenten in flüssigkristallinen Phasen für Displays, die auf dem ECB-Effekt beruhen.

Ähnliche Verbindungen sind z.B. aus der EP 111695-A für Displays, die nach dem Zwei-Frequenz-Verfahren arbeiten, bekannt. Weiterhin sind ähnliche Flüssigkristalle bekannt aus
Chem.Abs., Band 94, Nr.17, S.714, Zusammenfassung 139275c;
Chem.Abs., Band 107,Nr.16, S.709, Zusammenfassung 143003r;
Molecular Crystals and Liquid Crystals, Band 111, Nr.3-4, S.347-358 (1984);
Molecular Crystals and Liquid Crystals, Band 111, Nr.3-4, S.329-346 (1984); und
GB-A 2 155 465 .

Der Erfindung lag die Aufgabe zugrunde, neue stabile flüssigkristalline oder mesogene Verbindungen aufzufinden, die als Komponenten flüssigkristalliner Phasen geeignet sind. Diese Aufgabe wurde durch die

Bereitstellung der Verbindungen der Formel I gelöst.

Es wurde gefunden, daß die Verbindungen der Formel I als Komponenten flüssigkristalliner Phasen vorzüglich geeignet sind. Insbesondere sind mit ihrer Hilfe stabile flüssigkristalline Phasen mit relativ großer optischer Anisotropie und negativer dielektrischer Anisotropie herstellbar. Daher sind die Substanzen der Formel I bevorzugt für die Verwendung in Mischungen für ECB-Effekte geeignet.

Der ECB-Effekt (electrically controlled birefringence) oder auch DAP-Effekt (Deformation aufgerichteter Phasen) wurde erstmals 1971 beschrieben (M.F. Schieckel und K. Fahrenschon, "Deformation of nematic liquid crystals with vertical orientation in electrical fields", Appl. Phys.Lett. 19 (1971), 3912). Es folgten Arbeiten von J.F. Kahn (Appl.Phys.Lett. 20 (1972), 1193) und G. Labrunie und J. Robert (J.Appl.Phys. 44 - (1973), 4869).

Die Arbeiten von J. Robert und F. Clerc (SID 80 Digest Techn.Papers (1980), 30), J. Duchene (Displays 7 (1986), 3) und H. Schad (SID 82 Digest Techn. Papers (1982), 244) haben gezeigt, daß flüssigkristalline Phasen hohe Werte für das Verhältnis der elastischen Konstanten $K_3/K_1$, hohe Werte für die optische Anisotropie $\Delta n$ und negative Werte für die dielektrische Anisotropie $\Delta\epsilon$ aufweisen müssen, um für hochinformative Anzeigeelemente basierend auf dem ECB-Effekt eingesetzt werden zu können.

Auf dem ECB-Effekt basierende elektrooptische Anzeigeelemente weisen eine homöotrope Randorientierung auf, d.h. die flüssigkristalline Phase hat eine negative dielektrische Anisotropie.

Überraschend zeigte sich, daß der Zusatz von Verbindungen der Formel I flüssigkristalline Phasen liefert, die alle oben genannten Kriterien hervorragend erfüllen.

Mit der Bereitstellung der Verbindungen der Formel I wird außerdem ganz allgemein die Palette der flüssigkristallinen Substanzen, die sich unter verschiedenen anwendungstechnischen Gesichtspunkten zur Herstellung nematischer Gemische eignen, erheblich verbreitert.

Die Verbindungen der Formel I besitzen einen breiten Anwendungsbereich. In Abhängigkeit von der Auswahl der Substituenten können diese Verbindungen als Basismaterialien dienen, aus denen flüssigkristalline Phasen zum überwiegenden Teil zusammengesetzt sind; es können aber auch Verbindungen der Formel I flüssigkristallinen Basismaterialien aus anderen Verbindungsklassen zugesetzt werden, um beispielsweise die dielektrische und/oder optische Anisotropie eines solchen Dielektrikums zu optimieren. Die Verbindungen der Formel I eignen sich ferner als Zwischenprodukte zur Herstellung anderer Substanzen, die sich als Bestandteile flüssigkristalliner Phasen verwenden lassen.

Die Verbindungen der Formel I sind in reinem Zustand farblos und bilden flüssigkristalline Mesophasen in einem für die elektrooptische Verwendung günstig gelegenen Temperaturbereich. Chemisch, thermisch und gegen Licht sind sie sehr stabil.

Gegenstand der Erfindung sind somit die Verbindungen der Formel I sowie die Verwendung dieser Verbindungen als Komponenten flüssigkristalliner Phasen. Weiterhin sind Gegenstand der Erfindung flüssigkristalline Phasen mit einem Gehalt an mindestens einer Verbindung der Formel I oder einer Verbindung enthaltend den strukturellen Bestandteil

$-A^3-C{\equiv}C-A^4-$

worin $A^3$ und $A^4$ jeweils unabhängig voneinander einen

a) 1,4-Phenylen-, Pyridin-2,5-diyl- oder Pyrimidin-2,5-diyl-Rest,

b) trans-1,4-Cyclohexylenrest, bedeutet,

mit der Maßgabe, daß

eine und nur eine der Gruppen $A^3$ oder $A^4$ einen Pyridin- oder Pyrimidin-Rest bedeutet,

enthält, sie Flüssigkristall-Anzeigeelemente, die derartige Phasen enthalten.

Vor- und nachstehend haben $R^1$, $A^1$, $Z^1$, m, $A^3$, $A^4$, $Z^2$, $A^2$, n und $R^2$ die angegebene Bedeutung, sofern nicht ausdrücklich etwas anderes vermerkt ist.

Die Verbindungen der Formel I umfassen dementsprechend Verbindungen der Teilformeln Ia (mit zwei Ringen), Ib bis Ie (mit drei Ringen) und If bis Ii (mit vier Ringen):

$R^1-A^3-C{\equiv}C-A^4-R^2$   Ia

$R^1-A^3-C{\equiv}C-A^4-A^2-R^2$   Ib

$R^1-A^3-C{\equiv}C-A^4-Z^2-A^2-R^2$   Ic

$R^1-A^1-A^3-C{\equiv}C-A^4-R^2$   Id

$R^1$-$A^1$-$Z^1$-$A^3$-C≡C-$A^4$-$R^2$ Ie

$R^1$-$A^1$-$Z^1$-$A^3$-C≡C-$A^4$-$Z^2$-$A^2$-$R^2$ If

$R^1$-$A^1$-$A^3$-C≡C-$A^4$-$Z^2$-$A^2$-$R^2$ Ig

$R^1$-$A^1$-$Z^1$-$A^3$-C≡C-$A^4$-$A^2$-$R^2$ Ih

$R^1$-$A^1$-$A^3$-C≡C-$A^4$-$A^2$-$R^2$ Ii

Die bevorzugten Verbindungen der Teilformel Ia umfassen solche der Teilformeln Iaa bis Iax

$R^1$-Cyc-C≡C-Phe-$R^2$ Iaa

$R^1$-Cyc-C≡C-PheX-$R^2$ Iab

$R^1$-Phe-C = C-Pyd-$R^2$ Iaf

$R^1$-Phe-C≡C-Pyr-$R^2$ Iag

$R^1$-Cyc-C≡C-$A^4$-$R^2$ Ias

$R^1$-Pyd-C≡C-$A^4$-$R^2$ Iaw

$R^1$-Pyr-C≡C-$A^4$-$R^2$ Iax

Darunter sind diejenigen der Formeln Iab, Iaf, Ias und Iaw besonders bevorzugt.

Die bevorzugten Verbindungen der Teilformeln Ib, Ic, Id und Ie umfassen solche der Teilformeln I1 bis I16:

$R^1$-Cyc-C≡C-$A^4$-$Z^2$-$A^2$-$R^2$ I1

$R^1$-Pyd-C≡C-$A^4$-$Z^2$-$A^2$-$R^2$ I5

$R^1$-Pyr-C≡C-$A^4$-$Z^2$-$A^2$-$R^2$ I6

$R^1$-$A^3$-C≡C-Cyc-$Z^2$-$A^2$-$R^2$ I9

$R^1$-$A^3$-C≡C-Pyd-$Z^2$-$A^2$-$R^2$ I15

$R^1$-$A^3$-C≡C-Pyr-$Z^2$-$A^2$-$R^2$ I16

Darunter sind diejenigen der Formeln I1, I5, I6, I9 und I15 besonders bevorzugt.

Die bevorzugten Verbindungen der Teilformeln If, Ig, Ih und Ii umfassen solcher der Teilformeln I23, I27 und I28:

$R^1$-$A^1$-$Z^1$-Cyc-C≡C-$A^4$-$Z^2$-$A^2$-$R^2$ I23

$R^1$-$A^1$-$Z^1$-Pyd-C≡C-$A^4$-$Z^2$-$A^2$-$R^2$ I27

$R^1$-$A^1$-$Z^1$-Pyr-C≡C-$A^4$-$Z^2$-$A^2$-$R^2$ I28

In den vor- und nachstehenden Formeln bedeuten $R^1$ und $R^2$ vorzugsweise Alkyl, Alkoxy oder eine andere Oxaalkylgruppe.

Ferner bevorzugt für $R^1$ und $R^2$ sind Alkenylgruppen oder einfach durch -CN substituierte Alkylgruppen. Durch Halogen substituierte Alkylgruppen sind ebenfalls bevorzugt. Halogen bedeutet Fluor, Chlor oder Brom. Bevorzugt ist eine Substitution durch Fluor oder Chlor, insbesondere bevorzugt sind die Perfluoralkylgruppen wie Trifluormethyl, Pentafluorethyl oder Heptafluorpropyl.

Ferner stellt einer der Reste bevorzugt Halogen, -CN oder -NCS dar.

$A^1$ und $A^2$ sind bevorzugt Cyc, PheX oder Phe, PheX bedeutet vorzugsweise Monosubstitution durch F, Cl oder CN.

Weiterhin bevorzugt stellen $A^1$ und $A^2$ eine Pyd-, Dio- oder Pyr-Gruppe dar.

$A^3$ und $A^4$ bedeuten bevorzugt Cyc, Pyd, Pyr, Phe oder ferner bevorzugt PheX.

Eine der Gruppen $A^3$ oder $A^4$ ist Pyd, Pyr oder Cyc.

Nur eine der Gruppen $A^3$ oder $A^4$ bedeutet einen Pyridin-, Cyclohexylen- oder Pyrimidinrest.

m und n bedeuten jeweils unabhängig voneinander 0 oder 1. Vorzugsweise ist m + n = 1.

$Z^1$ und $Z^2$ bedeuten bevorzugt Einfachbindungen, -CO-O-oder -O-CO-. In zweiter Linie bevorzugt sind $-CH_2CH_2-$, -C≡C-, $-OCH_2-$ oder $-CH_2O-$.

Falls $R^1$ und/oder $R^2$ Alkylreste bedeuten, in denen auch eine ("Alkoxy" bzw. "Oxaalkyl") oder zwei ("Alkoxyalkoxy" bzw. "Dioxaalkyl") nicht benachbarte $CH_2$-Gruppen durch O-Atome ersetzt sein können, so können sie geradkettig oder verzweigt sein. Vorzugsweise sind sie geradkettig, haben 2, 3, 4, 5, 6 oder 7 C-Atome und bedeuten demnach bevorzugt Ethyl, Propyl, Butyl, Pentyl, Hexyl, Heptyl, Ethoxy, Propoxy, Butoxy, Pentoxy, Hexoxy, Heptoxy, 2-Oxapropyl (= Methoxymethyl), 2- (= Ethoxymethyl) oder 3-Oxabutyl (= 2-Methoxyethyl), 2-, 3- oder 4-Oxapentyl, 2-, 3-, 4- oder 5-Oxahexyl, 2-, 3-, 4-, 5- oder 6-Oxaheptyl, ferner Methyl, Octyl, Nonyl, Decyl, Undecyl, Dodecyl, Tridecyl, Tetradecyl, Pentadecyl, Methoxy, Octoxy, Nonoxy, Decoxy, Undecoxy, Dodecoxy, Tridecoxy, Tetradecoxy, Pentadecoxy, 2-, 3-, 4-, 5-, 6- oder 7-Oxaoctyl, 2-, 3-, 4-, 5-, 6-, 7- oder 8-Oxanonyl, 2-, 3-, 4-, 5-, 6-, 7-, 8- oder 9-Oxadecyl, 1,3-Dioxabutyl (= Methoxymethoxy), 1,3-, 1,4- oder 2,4-Dioxapentyl, 1,3-, 1,4-, 1,5-, 2,4-, 2,5- oder 3,5-Dioxahexyl, 1,3-, 1,4-, 1,5-, 1,6-, 2,4-, 2,5-, 2,6-, 3,5-, 3,6- oder 4,6-Dioxaheptyl.

Besonders bevorzugt sind auch Flügelgruppen in denen eine $CH_2$-Gruppe durch eine -C≡C-Gruppe ersetzt ist.

Verbindungen der Formel I mit verzweigten Flügelgruppen $R^1$ oder $R^2$ können gelegentlich wegen einer besseren Löslichkeit in den üblichen flüssigkristallinen Basismaterialien von Bedeutung sein, insbesondere aber als chirale Dotierstoffe, wenn sie optisch aktiv sind.

Verzweigte Gruppen dieser Art enthalten in der Regel nicht mehr als eine Kettenverzweigung. Bevorzugte verzweigte Rest sind Isopropyl, 2-Butyl (= 1-Methylpropyl), Isobutyl (= 2-Methylpropyl), 2-Methylbutyl, Isopentyl (= 3-Methylbutyl), 2-Methylpentyl, 3-Methylpentyl, 2-Ethylhexyl, 2-Propylpentyl, 2-Octyl, Isopropoxy, 2-Methylpropoxy, 2-Methylbutoxy, 3-Methylbutoxy, 2-Methylpentoxy, 3-Methylpentoxy, 2-Ethylhexoxy, 1-Methylhexoxy, 1-Methylheptoxy (= 2-Octyloxy), 2-Oxa-3-methylbutyl, 3-Oxa-4-methylpentyl, 4-Methylhexyl, 2-Nonyl, 2-Decyl, 2-Dodecyl, 6-Methyloctoxy, 6-Methyloctanoyloxy, 5-Methylheptyloxycarbonyl, 2-Methylbutyryloxy, 3-Methylvaleryloxy, 4-Methylhexanoyloxy, 2-Chlorpropionyloxy, 2-Chlor-3-methylbutyryloxy, 2-Chlor-4-methylvaleryloxy, 2-Chlor-3-methylvaleryloxy, 2-Methyl-3-oxapentyl, 2-Methyl-3-oxahexyl.

Bei Verbindungen mit verzweigten Flügelgruppen umfaßt Formel I sowohl die optischen Antipoden als auch Racemate sowie deren Gemische.

Unter den Verbindungen der Formel I und deren Unterformeln sind diejenigen bevorzugt, in denen mindestens einer der darin enthaltenen Reste eine der angegebenen bevorzugten Bedeutungen hat.

Bei m + n = 0 und $A^3$ = trans-1,4-Cyclohexylengruppe bedeutet $A^4$ Phe oder PheX.

Ferner sind Verbindungen der Formel I bevorzugt, worin $R^2$ Halogen bedeutet, m + n = 0, $A^3$ eine trans-1,4-Cyclohexylen- und $A^4$ eine unsubstituierte 1,4-Phenylengruppe ist.

Besonders bevorzugte kleinere Gruppen von Verbindungen sind diejenigen der Formeln 1 bis 50:

Alkyl-Cyc-C≡C-Phe-Halogen 1

Alkyl-Cyc-C≡C-PheX-Alkyl 2

Alkyl-Cyc-C≡C-PheX-CN 3

Alkyl-Cyc-C≡C-PheX-Halogen 4

Alkyl-Phe-C≡C-Pyd-Alkyl 7

Alkyl-Pyd-C≡C-Phe-Halogen 10

Alkoxy-Pyd-C≡C-Phe-Alkyl 11

Alkyl-Pyd-C≡C-Phe-Alkoxy 12

Alkyl-Phe-C≡C-Pyn-Alkyl 14

Alkyl-Cyc-Phe-C≡C-Pyd-Alkyl 16

Alkyl-Cyc-C≡C-Phe-COO-Phe-$R^2$ 17

$R^1$-Cyc-$CH_2CH_2$-Cyc-C≡C-Phe-$R^2$ 21

$R^1$-Phe-Pyd-C≡C-Phe-$R^2$ 22

$R^1$-Pyr-C≡C-Phe-OCO-Phe-$R^2$ 23

$R^1$-Cyc-C≡C-PheX-COO-Cyc-$R^2$ 26

$R^1$-Phe-$CH_2CH_2$-Phe-C≡C-Pyd-$R^2$ 27

$R^1$-Phe-Phe-C≡C-Cyc-Phe-$R^2$ 28

$R^1$-Phe-COO-Phe-C≡C-Pyd-Phe-$R^2$ 29

$R^1$-Cyc-C≡C-Phe-Dio-$R^2$ 30

$R^1$-Phe-$OCH_2$-Phe-C≡C-Cyc-Phe-$R^2$ 31

Alkyl-Cyc-C≡C-Phe-C≡C-Pyd-Alkyl 44

Alkyl-Cyc-C≡C-Cyc-C≡C-Phe-Alkyl 45

Alkyl-Cyc-Cyc-C≡C-Phe-$CF_3$ 47

Alkyl-Phe-Cyc-C≡C-Phe-$CF_3$ 48

Alkyl-Cyc-C≡C-Phe-$C_2F_5$ 49

Alkyl-Cyc-C≡C-Phe-$CF_3$ 50

Bevorzugt sind auch Verbindungen der Formel I, in denen eine der Gruppen $A^1$, $A^2$, $A^3$ und $A^4$ eine 2,3-Dihalogen-1,4-phenylengruppe darstellt. Halogen bedeutet darin Fluor, Chlor oder Brom. Bevorzugter Substituent ist Fluor.

Besonders bevorzugte Verbindungen mit einer 2,3-Difluor-1,4-phenylengruppe sind diejenigen der Formeln 51 bis 64:

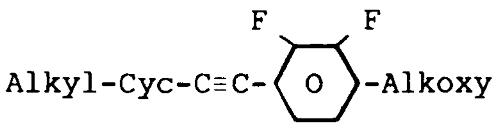

51

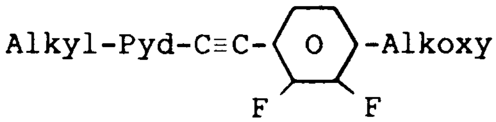

52

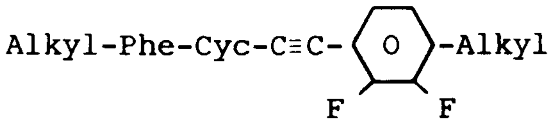

53

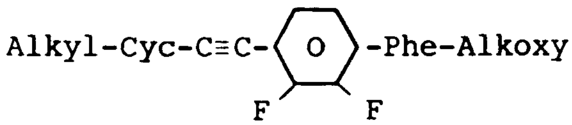

54

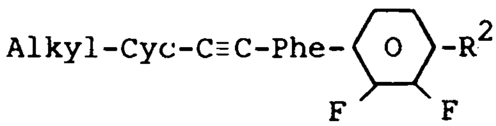

55

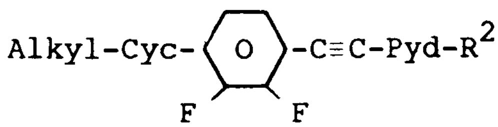

56

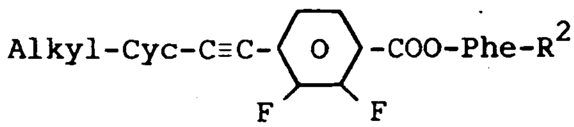

57

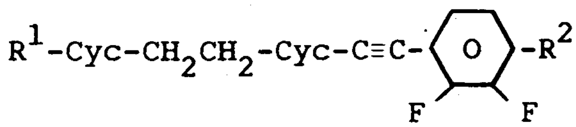

58

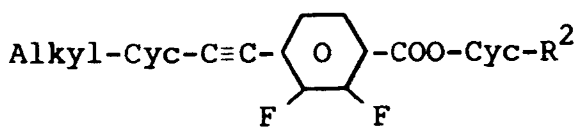

59

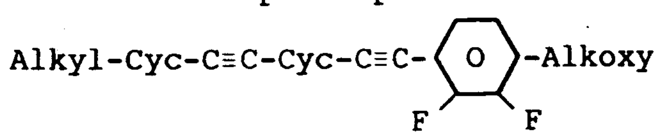

60

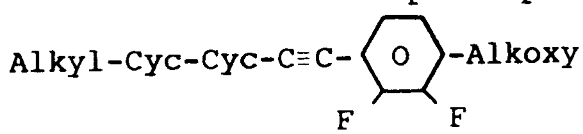

62

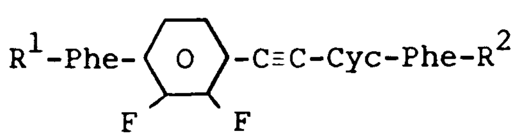

64

Die Verbindungen der Formel I werden nach an sich bekannten Methoden hergestellt, wie sie in der Literatur (z.B. in den Standardwerken wie Houben-Weyl, Methoden der Organischen Chemie, Georg-Thieme-Verlag, Stuttgart) beschrieben sind, und zwar unter Reaktionsbedingungen, die für die genannten Umsetzungen bekannt und geeignet sind. Dabei kann man auch von an sich bekannten, hier nicht näher erwähnten Varianten Gebrauch machen.

Die Ausgangsstoffe können gewünschtenfalls auch in situ gebildet werden, derart, daß man sie aus dem Reaktionsgemisch nicht isoliert, sondern sofort weiter zu den Verbindungen der Formel I umsetzt.

So können die Verbindungen der Formel I hergestellt werden, indem man die entsprechenden Stilbene bromiert und anschließend einer Dehydrohalogenierung unterwirft. Dabei kann man an sich bekannte, hier nicht näher erwähnte Varianten dieser Umsetzung anwenden.

Die Stilbene können hergestellt werden durch Umsetzung eines 4-substituierten Benzaldehyds mit einem entsprechenden Phosphorylid nach Wittig oder durch Umsetzung von einem 4-substituierten Phenylethylen mit einem entsprechenden Brombenzolderivat nach Heck.

Eine weitere Möglichkeit zur Herstellung der C-C-Dreifachbindung besteht darin, eine Verbindung, die sonst der Formel I entspricht, aber an Stelle der -C≡C-Bindung eine $-CH_2-CO-$Gruppe enthält, entweder mit einem anorganischen Säurechlorid umzusetzen, und die dann entstandene Gruppe $-CH_2-CCl_2-$ in Gegenwart einer Base zu dehydrohalogenieren, oder mit Semicarbazid und Selendioxid umzusetzen. Anschließend wird in Gegenwart von Methyllithium unter Erwärmen die Dreifachbindung eingeführt.

Ferner besteht die Möglichkeit, ein entsprechendes Benzilderivat mit Hydrazin und anschließend mit HgO in das Ethinderivat umzuwandeln.

Verbindungen der Formel I können auch hergestellt werden über die Kopplung von Alkinyl-Zink-Verbindungen mit Arylhalogeniden analog dem von A.O. King, E. Negishi, F.J. Villani und A. Silveira in J.Org.Chem. 43 (1978) 358 beschriebenen Verfahren.

Verbindungen der Formel I können auch über die Fritsch-Buttenberg-Wiechell-Umlagerung (Ann. 279, 319, 327, 332, 1894) hergestellt werden, bei der 1,1-Diaryl-2-halogenethylene umgelagert werden zu Diarylacetylenen in Gegenwart starker Basen.

Verbindungen der Formel I können weiterhin hergestellt werden aus 4-substituierten Phenyl- oder Cyclohexylacetylenen und Arylhalogeniden in Gegenwart eines Palladiumkatalysators, z.B. Bis-(triphenylphosphin)-palladium(II)-chlorid, und Kupfer(I)-jodid (beschrieben in Synthesis (1980) 627 oder Tetrahedron Letters 27 (1986) 1171).

Verbindungen der Formel I sind weiterhin erhältlich, indem man an ein entsprechendes Cyclohexenderivat eine Verbindung der Formel HX (Fluor-, Chlor-, Brom- oder Cyanwasserstoff) anlagert.

Diese Anlagerung gelingt z.B. in Gegenwart eines inerten Lösungsmittels, z.B. eines halogenierten Kohlenwasserstoffs wie $CH_2Cl_2$ oder $CHCl_3$, eines Nitrils wie Acetonitril oder eines Amids wie Dimethylformamid (DMF) bei Temperaturen zwischen etwa -10 und +150° und Drucken zwischen etwa 1 und 100 bar. Ein Zusatz von Katalysatoren kann günstig sein, z.B. kann eine HCN-Anlagerung durch Zusatz von Palladium-bis-[2,3-O-isopropyliden-2,3-dihydroxy-1,4-bis-(diphenylphosphino)-butan] katalysiert werden.

Ester der Formel I (-CO-O- oder -O-CO-Gruppe in $R^1$ und/oder $R^2$ und/oder $Z^2$ und/oder $Z^1$ = -CO-O- oder -O-CO-) können auch durch Veresterung entsprechender Carbonsäuren (oder ihrer reaktionsfähigen Derivate) mit Alkoholen bzw. Phenolen (oder ihren reaktionsfähigen Derivaten) erhalten werden. Die Veresterung von Säuren mit Alkoholen bzw. Phenolen kann auch mit DCC/DMAP durchgeführt werden.

Als reaktionsfähige Derivate der genannten Carbonsäuren eignen sich insbesondere die Säurehalogenide, vor allem die Chloride und Bromide, ferner die Anhydride, z.B. auch gemischte Anhydride, Azide oder Ester, insbesondere Alkylester mit 1-4 C-Atomen in der Alkylgruppe.

Als reaktionsfähige Derivate der genannten Alkohole bzw. Phenole kommen insbesondere die entsprechenden Metallalkoholate bzw. Phenolate in Betracht. Darin ist das Metall vorzugsweise ein Alkalimetall wie Na oder K.

Die Veresterung wird vorteilhaft in Gegenwart eines inerten Lösungsmittels durchgeführt. Gut geeignet sind insbesondere Ether wie Diethylether, Di-n-butylether, THF, Dioxan oder Anisol, Ketone wie Aceton, Butanon oder Cyclohexanon, Amide wie DMF oder Phosphorsäurehexamethyltriamid, Kohlenwasserstoffe wie Benzol, Toluol oder Xylol, Halogenkohlenwasserstoffe wie Tetrachlorkohlenstoff oder Tetrachlorethylen und Sulfoxide wie Dimethylsulfoxid oder Sulfolan. Mit Wasser nicht mischbare Lösungsmittel können gleichzeitig vorteilhaft zum azeotropen Abdestillieren des bei der Veresterung gebildeten Wassers verwendet werden. Gelegentlich kann auch ein Überschuß einer organischen Base, z.B. Pyridin, Chinolin oder Triethylamin als Lösungsmittel für die Veresterung angewandt werden. Die Veresterung kann auch in Abwesenheit eines Lösungsmittels, z.B. durch einfaches Erhitzen der Komponenten in Gegenwart von Natriumacetat, durchgeführt werden. Die Reaktionstemperatur liegt gewöhnlich zwischen -50° und +250°, vorzugsweise zwischen -20° und +80°. Bei diesen Temperaturen sind die Veresterungsreaktionen in der Regel nach 15 Minuten bis 48 Stunden beendet.

Im einzelnen hängen die Reaktionsbedingungen für die Veresterung weitgehend von der Natur der verwendeten Ausgangsstoffe ab. So wird eine freie Carbonsäure mit einem freien Alkohol oder Phenol in der Regel in Gegenwart einer starken Säure, beispielsweise einer Mineralsäure wie Salzsäure oder Schwefelsäure, umgesetzt. Eine bevorzugte Reaktionsweise ist die Umsetzung eines Säureanhydrids oder insbesondere eines Säurechlorids mit einem Alkohol, vorzugsweise in einem basischen Milieu, wobei als Basen insbesondere Alkalimetallhydroxide wie Natrium- oder Kaliumhydroxid, Alkalimetallcarbonate bzw.

-hydrogencarbonate wie Natriumcarbonat, Natriumhydrogencarbonat, Kaliumcarbonat oder Kaliumhydrogencarbonat, Alkalimetallacetate wie Natrium- oder Kaliumacetat, Erdalkalimetallhydroxide wie Calciumhydroxid oder organische Basen wie Triethylamin, Pyridin, Lutidin, Kollidin oder Chinolin von Bedeutung sind. Eine weitere bevorzugte Ausführungsform der Veresterung besteht darin, daß man den Alkohol bzw. das Phenol zunächst in das Natrium- oder Kaliumalkoholat bzw. -phenolat überführt, z.B. durch Behandlung mit ethanolischer Natron- oder Kalilauge, dieses isoliert und zusammen mit Natriumhydrogencarbonat oder Kaliumcarbonat unter Rühren in Aceton oder Diethylether suspendiert und diese Suspension mit einer Lösung des Säurechlorids oder Anhydrids in Diethylether, Aceton oder DMF versetzt, zweckmäßig bei Temperaturen zwischen etwa -25° und +20°.

Dioxanderivate bzw. Dithianderivate der Formel I (worin eine der Gruppen $A^1$ und/oder $A^2$ eine 1,3-Dioxan-2,5-diyl-Gruppe bzw. 1,3-Dithian-2,5-diyl-Gruppe bedeutet) werden zweckmäßig durch Reaktion eines entsprechenden Aldehyds (oder eines seiner reaktionsfähigen Derivate) mit einem entsprechenden 1,3-Diol (oder einem seiner reaktionsfähigen Derivate) bzw. einem entsprechenden 1,3-Dithiol hergestellt, vorzugsweise in Gegenwart eines inerten Lösungsmittels wie Benzol oder Toluol und/oder eines Katalysators, z.B. einer starken Säure wie Schwefelsäure, Benzol- oder p-Toluolsulfonsäure, bei Temperaturen zwischen etwa 20° und etwa 150°, vorzugsweise zwischen 80° und 120°. Als reaktionsfähige Derivate der Ausgangsstoffe eignen sich in erster Linie Acetale.

Die genannten Aldehyde und 1,3-Diole bzw. 1,3-Dithiole sowie ihre reaktionsfähigen Derivate sind zum Teil bekannt, zum Teil können sie ohne Schwierigkeiten nach Standardverfahren der organischen Chemie aus literaturbekannten Verbindungen hergestellt werden. Beispielsweise sind die Aldehyde durch Oxydation entsprechender Alkohole oder durch Reduktion entsprechender Carbonsäuren oder ihrer Derivate, die Diole durch Reduktion entsprechender Diester und die Dithiole durch Umsetzung entsprechender Dihalogenide mit NaSH erhältlich.

Zur Herstellung von Nitrilen der Formel I (worin $R^1$ oder $R^2$ CN bedeuten und/oder worin $A^3$ und/oder $A^4$ und/oder $A^1$ und/oder $A^2$ durch mindestens eine CN-Gruppe substituiert ist) können entsprechende Säureamide dehydratisiert werden. Die Amide sind z.B. aus entsprechenden Estern oder Säurehalogeniden durch Umsetzung mit Ammoniak erhältlich. Als wasserabspaltende Mittel eignen sich beispielsweise anorganische Säurechloride wie $SOCl_2$, $PCl_3$, $PCl_5$, $POCl_3$, $SO_2Cl_2$, $COCl_2$, ferner $P_2O_5$, $P_2S_5$,$AlCl_3$ (z.B. als Doppelverbindung mit NaCl), aromatische Sulfonsäuren und Sulfonsäurehalogenide. Man kann dabei in Gegenwart oder Abwesenheit eines inerten Lösungsmittels bei Temperaturen zwischen etwa 0° und 150° arbeiten; als Lösungsmittel kommen z.B. Basen wie Pyridin oder Triethylamin, aromatische Kohlenwasserstoffe wie Benzol, Toluol oder Xylol oder Amide wie DMF in Betracht.

Zur Herstellung der vorstehend genannten Nitrile der Formel I kann man auch entsprechende Säurehalogenide, vorzugsweise die Chloride, mit Sulfamid umsetzen, zweckmäßig in einem inerten Lösungsmittel wie Tetramethylensulfon bei Temperaturen zwischen etwa 80° und 150°, vorzugsweise bei 120°. Nach üblicher Aufarbeitung kann man direkt die Nitrile isolieren.

Ether der Formel I (worin $R^1$ und/oder $R^2$ eine Alkylgruppe bedeutet, worin eine oder zwei $CH_2$-Gruppen durch O-Atome ersetzt sind, und/oder worin $Z^2$ und/oder $Z^1$ eine $-OCH_2-$ oder eine $-CH_2O$-Gruppe ist) sind durch Veretherung entsprechender Phenole, erhältlich, wobei die Hydroxyverbindung zweckmäßig zunächst in ein entsprechendes Metallderivat, z.B. durch Behandeln mit NaH, $NaNH_2$, NaOH, KOH, $Na_2CO_3$ oder $K_2CO_3$ in das entsprechende Alkalimetallalkoholat oder Alkalimetallphenolat übergeführt wird. Dieses kann dann mit dem entsprechenden Alkylhalogenid, -sulfonat oder Dialkylsulfat umgesetzt werden, zweckmäßig in einem inerten Lösungsmittel wie Aceton, 1,2-Dimethoxyethan, DMF oder Dimethylsulfoxid oder auch einem Überschuß an wäßriger oder wäßrigalkoholischer NaOH oder KOH bei Temperaturen zwischen etwa 20° und 100°.

Die Thioether werden nach an sich bekannten Methoden hergestellt, wie sie in der Literatur (z.B. in den Standardwerken wie Houben-Weyl, Methoden der Organischen Chemie, Georg-Thieme-Verlag, Stuttgart) beschrieben sind, und zwar unter Reaktionsbedingungen, die für die genannten Umsetzungen bekannt und geeignet sind. Vorzugsweise werden die Thioether durch Behandlung entsprechender Halogenverbindungen, worin Halogen, Chlor, Brom oder Jod bedeutet, mit Salzen entsprechender Mercaptane erhalten.

Diese Halogenverbindungen sind entweder bekannt, oder sie können ohne Schwierigkeiten nach an sich bekannten Methoden in Analogie zu bekannten Verbindungen hergestellt werden. So sind beispielsweise p-substituierte Halogenbenzolderivate durch Halogenierung der entsprechenden Benzolderivate zugänglich. 4-substituierte Cyclohexylhalogenide sind beispielsweise durch Reduktion der entsprechenden 4-substituierten Cyclohexanone zu den 4-substituierten Cyclohexanolen und nachfolgender Substitution durch Halogenid erhältlich.

Bei der Synthese der Halogenverbindungen können im Prinzip alle Methoden angewendet werden, die für die Verbindungen bekannt sind, die an Stelle des Halogens andere Substituenten tragen. Der Fachmann

kann die erforderlichen Synthesevarianten nach Routinemethoden ableiten.

Zur Herstellung von Nitrilen der Formel I (worin $R^1$ oder $R^2$ CN bedeuten und/oder worin $A^3$ und/oder $A^4$ und/oder $A^1$ und/oder $A^2$ durch mindestens eine CN-Gruppe substituiert ist) können auch entsprechende Chlor- oder Bromverbindungen der Formel I mit einem Cyanid umgesetzt werden, zweckmäßig mit einem Metallcyanid wie NaCN, KCN oder $Cu_2(CN)_2$, z.B. in Gegenwart von Pyridin in einem inerten Lösungsmittel wie DMF oder N-Methylpyrrolidon bei Temperaturen zwischen 20° und 200°.

Die erfindungsgemäßen flüssigkristallinen Phasen bestehen aus vorzugsweise 3 bis 12 Komponenten, darunter mindestens einer Verbindung der Formel I. Die anderen Bestandteile werden vorzugsweise ausgewählt aus den nematischen oder nematogenen Substanzen, insbesondere den bekannten Substanzen, aus den Klassen der Azoxybenzole, Benzylidenaniline, Biphenyle, Terphenyle, Phenyl- oder Cyclohexylbenzoate, Cyclohexan-carbonsäurephenyl- oder -cyclohexyl-ester, Phenylcyclohexane, Cyclohexylbiphenyle, Cyclohexylcyclohexane, Cyclohexylnaphthaline, 1,4-Bis-cyclohexylbenzole, 4,4'-Bis-cyclohexylbiphenyle, Phenyl- oder Cyclophexylpyrimidine, Phenyl- oder Cyclohexyldioxane, Phenyl- oder Cyclohexyl-1,3-dithiane, 1,2-Diphenylethane, 1,2-Dicyclohexylethane, 1-Phenyl-2-cyclohexylethane, gegebenenfalls halogenierten Stilbene, Benzylphenylether und substituierten Zimtsäuren.

Die wichtigsten als Bestandteile derartiger flüssigkristalliner Phasen in Frage kommenden Verbindungen lassen sich durch die Formel IV charakterisieren,

$R^6$-L-G-E-$R^7$ IV

worin L und E je ein carbo- oder heterocyclisches Ringsystem aus der aus 1,4-disubstituierten Benzol- und Cyclohexanringen, 4,4'-disubstituierten Biphenyl-, Phenylcyclohexan- und Cyclohexylcyclohexansystemen, 2,5-disubstituierten Pyrimidin- und 1,3-Dioxanringen, 2,6-disubstituiertem Naphthalin, Di- und Tetrahydronaphthalin, Chinazolin und Tetrahydrochinazolin gebildeten Gruppe,

| | | |
|---|---|---|
| G | -CH = CH- | -N(O) = N- |
| | -CH = CY- | -CH = N(O)- |
| | -C≡C- | $-CH_2-CH_2-$ |
| | -CO-O- | $-CH_2-O-$ |
| | -CO-S- | $-CH_2-S-$ |
| | -CH = N- | -COO-Phe-COO- |

oder eine C-C-Einfachbindung,

Y Halogen, vorzugsweise Chlor, oder -CN, und

$R^6$ und $R^7$ Alkyl, Alkoxy, Alkanoyloxy oder Alkoxycarbonyloxy mit bis zu 18, vorzugsweise bis zu 8 Kohlenstoffatomen, oder einer dieser Reste durch CN, NC, $NO_2$, $CF_3$, F, Cl oder Br bedeuten.

Bei den meisten dieser Verbindungen sind $R^6$ und $R^7$ voneinander verschieden, wobei einer dieser Reste meist eine Alkyl- oder Alkoxygruppe ist. Auch auch andere Varianten der vorgesehenen Substituenten sind gebräuchlich. Viele solcher Substanzen oder auch Gemische davon sind im Handel erhältlich. Alle diese Substanzen sind nach literaturbekannten Methoden erhältlich.

Die erfindungsgemäßen Phasen enthalten etwa 0,1 bis 99 vorzugsweise 10 bis 95 %, einer oder mehrer Verbindungen der Formel I. Weiterhin bevorzugt sind erfindungsgemäße flüssigkristalline Phasen, enthaltend 0,1-40, vorzugsweise 0,5-30 % einer oder mehrerer Verbindungen der Formel I.

Die Verbindungen der Formel I können auch als Komponenten smektischer oder chiral getilteter smektischer flüssigkristalliner Phasen verwendet werden. Diese Phasen sind bevorzugt chiral getiltete smektische flüssigkristalline Phasen, deren achirale Basismischung neben Verbindungen der Formel I mindestens eine andere Komponente mit negativer oder betragsmäßig kleiner positiver dielektrischen Anisotropie enthält. Diese weitere(n) Komponente(n) der achiralen Basismischung kann (können) zu 1 bis 50 %, vorzugsweise 10 bis 25 %, der Basismischung ausmachen.

Die Herstellung der erfindungsgemäßen Phasen erfolgt in an sich üblicher Weise. In der Regel werden die Komponenten ineinander gelöst, zweckmäßig bei erhöhter Temperatur.

Durch geeignete Zusätze können die flüssigkristallinen Phasen nach der Erfindung so modifiziert werden, daß sie in allen bisher bekannt gewordenen Arten von Flüssigkristallanzeigeelementen verwendet werden können.

Derartige Zusätze sind dem Fachmann bekannt und in der Literatur ausführlich beschrieben. Beispielsweise können Leitsalze, vorzugsweise Ethyl-dimethyl-dodecyl-ammonium-4-hexyloxybenzoat, Tetrabutylammonium-tetraphenylboranat oder Komplexsalze von Kronenethern (vg. z.B. I. Haller et al., Mol.

Cryst. Lig. Cryst. Band 24, Seiten 249-258 (1973)) zur Verbesserung der Leitfähigkeit, dichroitische Farbstoffe zur Herstellung farbiger Guest-Host-Systeme oder Substanzen zur Veränderung der dielektrischen Anisotropie, der Viskosität und/oder der Orientierung der nematischen Phasen zugesetzt werden. Derartige Substanzen sind z.B. in den DE-OS 22 09 127, 22 40 864, 23 21 632, 23 38 281, 24 50 088, 26 37 430, 28 53 728 und 29 02 177 beschrieben.

Die folgenden Beispiele sollen die Erfindung erläutern, ohne sie zu begrenzen. F. = Schmelzpunkt, K. = Klärpunkt. Vor- und nachstehend bedeuten Prozentangaben Gewichtsprozent; alle Temperaturen sind in Grad Celsius angegeben. "Übliche Aufarbeitung" bedeutet: man gibt Wasser hinzu, extrahiert mit Methylenchlorid, trennt ab, trocknet die organische Phase, dampft ein und reinigt das Produkt durch Kristallisation und/oder Chromatographie.

Beispiel 1

Zu einem Gemisch aus 0,01 mol 2-Fluor-4-brombenzonitril, 0,01 mol 4-Pentylcyclohexylacetylen (herstellbar z.B. nach Corey, Fuchs in Tetrahedron Letters (1972) 3769 aus dem entsprechenden Cyclohexancarbaldehyd) und 40 ml Triethylamin gibt man bei Raumtemperatur 0,2 mmol Bis-(triphenylphosphin)-palladium(II)-chlorid und 0,1 mmol Kupfer(I)-jodid und rührt 12 Stunden. Die Reaktion läßt sich mit Hilfe der Dünnschichtchromatographie verfolgen. Nach beendeter Reaktion wird die Suspension filtriert und das Filtrat eingedampft. Nach Reinigung durch Chromatographie und/oder Kristallisation erhält man 1-(trans-4-Pentylcyclohexyl)-2-(3-fluor-4-cyanphenyl)-acetylen mit F. = 38° und K. = 40.3°.

Analog werden hergestellt:

1-(trans-4-Methylcyclohexyl)-2-(3-fluor-4-cyanphenyl)-acetylen
1-(trans-4-Ethylcyclohexyl)-2-(3-fluor-4-cyanphenyl)-acetylen
1-(trans-4-Propylcyclohexyl)-2-(3-fluor-4-cyanphenyl)-acetylen
1-(trans-4-Butylcyclohexyl)-2-(3-fluor-4-cyanphenyl)-acetylen
1-(trans-4-Hexylcyclohexyl)-2-(3-fluor-4-cyanphenyl)-acetylen
1-(trans-4-Heptylcyclohexyl)-2-(3-fluor-4-cyanphenyl)-acetylen
1-(trans-4-Methylcyclohexyl)-2-(3-chlor-4-cyanphenyl)-acetylen
1-(trans-4-Ethylcyclohexyl)-2-(3-chlor-4-cyanphenyl)-acetylen
1-(trans-4-Propylcyclohexyl)-2-(3-chlor-4-cyanphenyl)-acetylen
1-(trans-4-Butylcyclohexyl)-2-(3-chlor-4-cyanphenyl)-acetylen
1-(trans-4-Pentylcyclohexyl)-2-(3-chlor-4-cyanphenyl)-acetylen
1-(trans-4-Hexylcyclohexyl)-2-(3-chlor-4-cyanphenyl)-acetylen
1-(trans-4-Heptylcyclohexyl)-2-(3-chlor-4-cyanphenyl)-acetylen
1-(trans-4-Methylcyclohexyl)-2-(4-fluorphenyl)-acetylen
1-(trans-4-Ethylcyclohexyl)-2-(4-fluorphenyl)-acetylen
1-(trans-4-Propylcyclohexyl)-2-(4-fluorphenyl)-acetylen
1-(trans-4-Butylcyclohexyl)-2-(4-fluorphenyl)-acetylen
1-(trans-4-Pentylcyclohexyl)-2-(4-fluorphenyl)-acetylen
1-(trans-4-Hexylcyclohexyl)-2-(4-fluorphenyl)-acetylen
1-(trans-4-Heptylcyclohexyl)-2-(4-fluorphenyl)-acetylen
1-(trans-4-Octylcyclohexyl)-2-(4-fluorphenyl)-acetylen
1-(trans-4-Nonylcyclohexyl)-2-(4-fluorphenyl)-acetylen
1-(trans-4-Decylcyclohexyl)-2-(4-fluorphenyl)-acetylen
1-(trans-4-Methylcyclohexyl)-2-(4-chlorphenyl)-acetylen
1-(trans-4-Ethylcyclohexyl)-2-(4-chlorphenyl)-acetylen
1-(trans-4-Propylcyclohexyl)-2-(4-chlorphenyl)-acetylen
1-(trans-4-Butylcyclohexyl)-2-(4-chlorphenyl)-acetylen
1-(trans-4-Pentylcyclohexyl)-2-(4-chlorphenyl)-acetylen
1-(trans-4-Hexylcyclohexyl)-2-(4-chlorphenyl)-acetylen
1-(trans-4-Heptylcyclohexyl)-2-(4-chlorphenyl)-acetylen
1-(trans-4-Octylcyclohexyl)-2-(4-chlorphenyl)-acetylen
1-(trans-4-Nonylcyclohexyl)-2-(4-chlorphenyl)-acetylen
1-(trans-4-Decylcyclohexyl)-2-(4-chlorphenyl)-acetylen
1-(trans-4-Methylcyclohexyl)-2-(2,4-difluorphenyl)-acetylen
1-(trans-4-Ethylcyclohexyl)-2-(2,4-difluorphenyl)-acetylen
1-(trans-4-Propylcyclohexyl)-2-(2,4-difluorphenyl)-acetylen
1-(trans-4-Butylcyclohexyl)-2-(2,4-difluorphenyl)-acetylen

1-(trans-4-Pentylcyclohexyl)-2-(2,4-difluorphenyl)-acetylen
1-(trans-4-Hexylcyclohexyl)-2-(2,4-difluorphenyl)-acetylen
1-(trans-4-Heptylcyclohexyl)-2-(2,4-difluorphenyl)-acetylen
1-(trans-4-Methylcyclohexyl)-2-(3,4-difluoryhenyl)-acetylen
1-(trans-4-Ethylcyclohexyl)-2-(3,4-difluorphenyl)-acetylen
1-(trans-4-Propylcyclohexyl)-2-(3,4-difluorphenyl)-acetylen
1-(trans-4-Butylcyclohexyl)-2-(3,4-difluorphenyl)-acetylen
1-(trans-4-Pentylcyclohexyl)-2-(3,4-difluorphenyl)-acetylen
1-(trans-4-Hexylcyclohexyl)-2-(3,4-difluorphenyl)-acetylen
1-(trans-4-Heptylcyclohexyl)-2-(3,4-difluorphenyl) - acetylen
1-[trans-4-(4-Methylphenyl)cyclohexyl]-2-(4-methoxyphenyl)-acetylen
1-[trans-4-(4-Ethylphenyl)cyclohexyl]-2-(4-methoxyphenyl)-acetylen
1-[trans-4-(4-Propylphenyl)cyclohexyl]-2-(4-methoxyphenyl)-acetylen
1-[trans-4-(4-Butylphenyl)cyclohexyl]-2-(4-methoxyphenyl)-acetylen
1-[trans-4-(4-Pentylphenyl)cyclohexyl]-2-(4-methoxyphenyl)-acetylen
1-[trans-4-(4-Hexylphenyl)cyclohexyl]-2-(4-methoxyphenyl)-acetylen
1-[trans-4-(4-Heptylphenyl)cyclohexyl]-2-(4-methoxyphenyl)-acetylen
1-[trans-4-(4-Methylphenyl)cyclohexyl]-2-(4-ethoxyphenyl)-acetylen
1-[trans-4-(4-Ethylphenyl)cyclohexyl]-2-(4-ethoxyphenyl)-acetylen
1-[trans-4-(4-Propylphenyl)cyclohexyl]-2-(4-ethoxyphenyl)-acetylen
1-[trans-4-(4-Butylphenyl)cyclohexyl]-2-(4-ethoxyphenyl)-acetylen
1-[trans-4-(4-Pentylphenyl)cyclohexyl]-2-(4-ethoxyphenyl)-acetylen
1-[trans-4-(4-Hexylphenyl)cyclohexyl]-2-(4-ethoxyphenyl)-acetylen
1-[trans-4-(4-Heptylphenyl)cyclohexyl]-2-(4-ethoxyphenyl)-acetylen
1-[trans-4-(4-Methylphenyl)cyclohexyl]-2-(4-propoxyphenyl)-acetylen
1-[trans-4-(4-Ethylphenyl)cyclohexyl]-2-(4-propoxyphenyl)-acetylen
1-[trans-4-(4-Propylphenyl)cyclohexyl]-2-(4-propoxyphenyl)-acetylen
1-[trans-4-(4-Butylphenyl)cyclohexyl]-2-(4-propoxyphenyl)-acetylen
1-[trans-4-(4-Pentylphenyl)cyclohexyl]-2-(4-propoxyphenyl)-acetylen
1-[trans-4-(4-Hexylphenyl)cyclohexyl]-2-(4-propoxyphenyl)-acetylen
1-[trans-4-(4-Heptylphenyl)cyclohexyl]-2-(4-propoxyphenyl)-acetylen
1-[trans-4-(4-Methylphenyl)cyclohexyl]-2-(4-butyloxyphenyl)-acetylen
1-[trans-4-(4-Ethylphenyl)cyclohexyl]-2-(4-butyloxyphenyl)-acetylen
1-[trans-4-(4-Propylphenyl)cyclohexyl]-2-(4-butyloxyphenyl)-acetylen
1-[trans-4-(4-Butylphenyl)cyclohexyl]-2-(4-butyloxyphenyl)-acetylen
1-[trans-4-(4-Pentylphenyl)cyclohexyl]-2-(4-butyloxyphenyl)-acetylen
1-[trans-4-(4-Hexylphenyl)cyclohexyl]-2-(4-butyloxyphenyl)-acetylen
1-[trans-4-(4-Heptylphenyl)cyclohexyl]-2-(4-butyloxyphenyl)-acetylen
1-[trans-4-(4-Methylphenyl)cyclohexyl]-2-(4-pentyloxyphenyl)-acetylen
1-[trans-4-(4-Ethylphenyl)cyclohexyl]-2-(4-pentyloxyphenyl)-acetylen
1-[trans-4-(4-Propylphenyl)cyclohexyl]-2-(4-pentyloxyphenyl)-acetylen
1-[trans-4-(4-Butylphenyl)cyclohexyl]-2-(4-pentyloxyphenyl)-acetylen
1-[trans-4-(4-Pentylphenyl)cyclohexyl]-2-(4-pentyloxyphenyl)-acetylen
1-[trans-4-(4-Hexylphenyl)cyclohexyl]-2-(4-pentyloxyphenyl)-acetylen
1-[trans-4-(4-Heptylphenyl)cyclohexyl]-2-(4-pentyloxyphenyl)-acetylen
1-[trans-4-(4-Methylphenyl)cyclohexyl]-2-(4-hexyloxyphenyl)-acetylen
1-[trans-4-(4-Ethylphenyl)cyclohexyl]-2-(4-hexyloxyphenyl)-acetylen
1-[trans-4-(4-Propylphenyl)cyclohexyl]-2-(4-hexyloxyphenyl)-acetylen
1-[trans-4-(4-Butylphenyl)cyclohexyl]-2-(4-hexyloxyphenyl)-acetylen
1-[trans-4-(4-Pentylphenyl)cyclohexyl]-2-(4-hexyloxyphenyl)-acetylen
1-[trans-4-(4-Hexylphenyl)cyclohexyl]-2-(4-hexyloxyphenyl)-acetylen
1-[trans-4-(4-Heptylphenyl)cyclohexyl]-2-(4-hexyloxyphenyl)-acetylen
1-[trans-4-(4-Methylphenyl)cyclohexyl]-2-(4-methylphenyl)-acetylen
1-[trans-4-(4-Ethylphenyl)cyclohexyl]-2-(4-methylphenyl)-acetylen
1-[trans-4-(4-Propylphenyl)cyclohexyl]-2-(4-methylphenyl)-acetylen
1-[trans-4-(4-Butylphenyl)cyclohexyl]-2-(4-methylphenyl)-acetylen
1-[trans-4-(4-Pentylphenyl)cyclohexyl]-2-(4-methylphenyl)-acetylen
1-[trans-4-(4-Hexylphenyl)cyclohexyl]-2-(4-methylphenyl)-acetylen

1-[trans-4-(4-Heptylphenyl)cyclohexyl]-2-(4-methylphenyl)-acetylen
1-[trans-4-(4-Octylphenyl)cyclohexyl]-2-(4-methylphenyl)-acetylen
1-[trans-4-(4-Nonylphenyl)cyclohexyl]-2-(4-methylphenyl)-acetylen
1-[trans-4-(4-Decylphenyl)cyclohexyl]-2-(4-methylphenyl)-acetylen
1-[trans-4-(4-Methylphenyl)cyclohexyl]-2-(4-ethylphenyl)-acetylen
1-[trans-4-(4-Ethylphenyl)cyclohexyl]-2-(4-ethylphenyl)-acetylen
1-[trans-4-(4-Propylphenyl)cyclohexyl]-2-(4-ethylphenyl)-acetylen
1-[trans-4-(4-Butylphenyl)cyclohexyl]-2-(4-ethylphenyl)-acetylen
1-[trans-4-(4-Pentylphenyl)cyclohexyl]-2-(4-ethylphenyl)-acetylen
1-[trans-4-(4-Hexylphenyl)cyclohexyl]-2-(4-ethylphenyl)-acetylen
1-[trans-4-(4-Heptylphenyl)cyclohexyl]-2-(4-ethylphenyl)-acetylen
1-[trans-4-(4-Methylphenyl)cyclohexyl]-2-(4-propylphenyl)-acetylen
1-[trans-4-(4-Ethylphenyl)cyclohexyl]-2-(4-propylphenyl)-acetylen
1-[trans-4-(4-Propylphenyl)cyclohexyl]-2-(4-propylphenyl)-acetylen
1-[trans-4-(4-Butylphenyl)cyclohexyl]-2-(4-propylphenyl)-acetylen
1-[trans-4-(4-Pentylphenyl)cyclohexyl]-2-(4-propylphenyl)-acetylen
1-[trans-4-(4-Hexylphenyl)cyclohexyl]-2-(4-propylphenyl)-acetylen
1-[trans-4-(4-Heptylphenyl)cyclohexyl]-2-(4-propylphenyl)-acetylen
1-[trans-4-(4-Methylphenyl)cyclohexyl]-2-(4-butylphenyl)-acetylen
1-[trans-4-(4-Ethylphenyl)cyclohexyl]-2-(4-butylphenyl)-acetylen
1-[trans-4-(4-Propylphenyl)cyclohexyl]-2-(4-butylphenyl)-acetylen
1-[trans-4-(4-Butylphenyl)cyclohexyl]-2-(4-butylphenyl)-acetylen
1-[trans-4-(4-Pentylphenyl)cyclohexyl]-2-(4-butylphenyl)-acetylen
1-[trans-4-(4-Hexylphenyl)cyclohexyl]-2-(4-butylphenyl)-acetylen
1-[trans-4-(4-Heptylphenyl)cyclohexyl]-2-(4-butylphenyl)-acetylen
1-[trans-4-(4-Methylphenyl)cyclohexyl]-2-(4-pentylphenyl)-acetylen
1-[trans-4-(4-Ethylphenyl)cyclohexyl]-2-(4-pentylphenyl)-acetylen
1-[trans-4-(4-Propylphenyl)cyclohexyl]-2-(4-pentylphenyl)-acetylen
1-[trans-4-(4-Butylphenyl)cyclohexyl]-2-(4-pentylphenyl)-acetylen
1-[trans-4-(4-Pentylphenyl)cyclohexyl]-2-(4-pentylphenyl)-acetylen
1-[trans-4-(4-Hexylphenyl)cyclohexyl]-2-(4-pentylphenyl)-acetylen
1-[trans-4-(4-Heptylphenyl)cyclohexyl]-2-(4-pentylphenyl)-acetylen
1-[trans-4-(4-Methylphenyl)cyclohexyl]-2-(4-hexylphenyl)-acetylen
1-[trans-4-(4-Ethylphenyl)cyclohexyl]-2-(4-hexylphenyl)-acetylen
1-[trans-4-(4-Propylphenyl)cyclohexyl]-2-(4-hexylphenyl)-acetylen
1-[trans-4-(4-Butylphenyl)cyclohexyl]-2-(4-hexylphenyl)-acetylen
1-[trans-4-(4-Pentylphenyl)cyclohexyl]-2-(4-hexylphenyl)-acetylen
1-[trans-4-(4-Hexylphenyl)cyclohexyl]-2-(4-hexylphenyl)-acetylen
1-[trans-4-(4-Heptylphenyl)cyclohexyl]-2-(4-hexylphenyl)-acetylen
1-[trans-4-(4-Methylphenyl)cyclohexyl]-2-(4-heptylphenyl)-acetylen
1-[trans-4-(4-Ethylphenyl)cyclohexyl]-2-(4-heptylphenyl)-acetylen
1-[trans-4-(4-Propylphenyl)cyclohexyl]-2-(4-heptylphenyl)-acetylen
1-[trans-4-(4-Butylphenyl)cyclohexyl]-2-(4-heptylphenyl)-acetylen
1-[trans-4-(4-Pentylphenyl)cyclohexyl]-2-(4-heptylphenyl)-acetylen
1-[trans-4-(4-Hexylphenyl)cyclohexyl]-2-(4-heptylphenyl)-acetylen
1-[trans-4-(4-Heptylphenyl)cyclohexyl]-2-(4-heptylphenyl)-acetylen
1-[trans-4-(4-Methylphenyl)cyclohexyl]-2-(4-octylphenyl)-acetylen
1-[trans-4-(4-Ethylphenyl)cyclohexyl]-2-(4-octylphenyl)-acetylen
1-[trans-4-(4-Propylphenyl)cyclohexyl]-2-(4-octylphenyl)-acetylen
1-[trans-4-(4-Butylphenyl)cyclohexyl]-2-(4-octylphenyl)-acetylen
1-[trans-4-(4-Pentylphenyl)cyclohexyl]-2-(4-octylphenyl)-acetylen
1-[trans-4-(4-Hexylphenyl)cyclohexyl]-2-(4-octylphenyl)-acetylen
1-[trans-4-(4-Heptylphenyl)cyclohexyl]-2-(4-octylphenyl)-acetylen
1-[trans-4-(4-Methylphenyl)cyclohexyl]-2-(3-fluor-4-propylphenyl)-acetylen
1-[trans-4-(4-Ethylphenyl)cyclohexyl]-2-(3-fluor-4-propylphenyl)-acetylen
1-[trans-4-(4-Propylphenyl)cyclohexyl]-2-(3-fluor-4-propylphenyl)-acetylen
1-[trans-4-(4-Butylphenyl)cyclohexyl]-2-(3-fluor-4-propylphenyl)-acetylen
1-[trans-4-(4-Pentylphenyl)cyclohexyl]-2-(3-fluor-4-propylphenyl)-acetylen

1-[trans-4-(4-Hexylphenyl)cyclohexyl]-2-(3-fluor-4-propylphenyl)-acetylen
1-[trans-4-(4-Heptylphenyl)cyclohexyl]-2-(3-fluor-4-propylphenyl)-acetylen
1-[trans-4-(4-Methylphenyl)cyclohexyl]-2-(3-fluor-4-butylphenyl)-acetylen
1-[trans-4-(4-Ethylphenyl)cyclohexyl]-2-(3-fluor-4-butylphenyl)-acetylen
1-[trans-4-(4-Propylphenyl)cyclohexyl]-2-(3-fluor-4-butylphenyl)-acetylen
1-[trans-4-(4-Butylphenyl)cyclohexyl]-2-(3-fluor-4-butylphenyl)-acetylen
1-[trans-4-(4-Pentylphenyl)cyclohexyl]-2-(3-fluor-4-butylphenyl)-acetylen
1-[trans-4-(4-Hexylphenyl)cyclohexyl]-2-(3-fluor-4-butylphenyl)-acetylen
1-[trans-4-(4-Heptylphenyl)cyclohexyl]-2-(3-fluor-4-butylphenyl)-acetylen
1-[trans-4-(4-Methylphenyl)cyclohexyl]-2-(3-fluor-4-pentylphenyl)-acetylen
1-[trans-4-(4-Ethylphenyl)cyclohexyl]-2-(3-fluor-4-pentylphenyl)-acetylen
1-[trans-4-(4-Propylphenyl)cyclohexyl]-2-(3-fluor-4-pentylphenyl)-acetylen
1-[trans-4-(4-Butylphenyl)cyclohexyl]-2-(3-fluor-4-pentylphenyl)-acetylen
1-[trans-4-(4-Pentylphenyl)cyclohexyl]-2-(3-fluor-4-pentylphenyl)-acetylen
1-[trans-4-(4-Hexylphenyl)cyclohexyl]-2-(3-fluor-4-pentylphenyl)-acetylen
1-[trans-4-(4-Heptylphenyl)cyclohexyl]-2-(3-fluor-4-pentylphenyl)-acetylen
1-[trans-4-(4-Octylphenyl)cyclohexyl]-2-(3-fluor-4-pentylphenyl)-acetylen
1-[trans-4-(4-Nonylphenyl)cyclohexyl]-2-(3-fluor-4-pentylphenyl)-acetylen
1-[trans-4-(4-Decylphenyl)cyclohexyl]-2-(3-fluor-4-pentylphenyl)-acetylen
1-[trans-4-(4-Methylphenyl)cyclohexyl]-2-(3-fluor-4-hexylphenyl)-acetylen
1-[trans-4-(4-Ethylphenyl)cyclohexyl]-2-(3-fluor-4-hexylphenyl)-acetylen
1-[trans-4-(4-Propylphenyl)cyclohexyl]-2-(3-fluor-4-hexylphenyl)-acetylen
1-[trans-4-(4-Butylphenyl)cyclohexyl]-2-(3-fluor-4-hexylphenyl)-acetylen
1-[trans-4-(4-Pentylphenyl)cyclohexyl]-2-(3-fluor-4-hexylphenyl)-acetylen
1-[trans-4-(4-Hexylphenyl)cyclohexyl]-2-(3-fluor-4-hexylphenyl)-acetylen
1-[trans-4-(4-Heptylphenyl)cyclohexyl]-2-(3-fluor-4-hexylphenyl)-acetylen
1-[trans-4-(4-Methylphenyl)cyclohexyl]-2-(3-chlor-4-propylphenyl)-acetylen
1-[trans-4-(4-Ethylphenyl)cyclohexyl]-2-(3-chlor-4-propylphenyl)-acetylen
1-[trans-4-(4-Propylphenyl)cyclohexyl]-2-(3-chlor-4-propylphenyl)-acetylen
1-[trans-4-(4-Butylphenyl)cyclohexyl]-2-(3-chlor-4-propylphenyl)-acetylen
1-[trans-4-(4-Pentylphenyl)cyclohexyl]-2-(3-chlor-4-propylphenyl)-acetylen
1-[trans-4-(4-Hexylphenyl)cyclohexyl]-2-(3-chlor-4-propylphenyl)-acetylen
1-[trans-4-(4-Heptylphenyl)cyclohexyl]-2-(3-chlor-4-propylphenyl)-acetylen
1-[trans-4-(4-Methylphenyl)cyclohexyl]-2-(3-cyan-4-propylphenyl)-acetylen
1-[trans-4-(4-Ethylphenyl)cyclohexyl]-2-(3-cyan-4-propylphenyl)-acetylen
1-[trans-4-(4-Propylphenyl)cyclohexyl]-2-(3-cyan-4-propylphenyl)-acetylen
1-[trans-4-(4-Butylphenyl)cyclohexyl]-2-(3-cyan-4-propylphenyl)-acetylen
1-[trans-4-(4-Pentylphenyl)cyclohexyl]-2-(3-cyan-4-propylphenyl)-acetylen
1-[trans-4-(4-Hexylphenyl)cyclohexyl]-2-(3-cyan-4-propylphenyl)-acetylen
1-[trans-4-(4-Heptylphenyl)cyclohexyl]-2-(3-cyan-4-propylphenyl)-acetylen
1-[trans-4-(4-Methylphenyl)cyclohexyl]-2-(3-chlor-4-butylphenyl)-acetylen
1-[trans-4-(4-Ethylphenyl)cyclohexyl]-2-(3-chlor-4-butylphenyl)-acetylen
1-[trans-4-(4-Propylphenyl)cyclohexyl]-2-(3-chlor-4-butylphenyl)-acetylen
1-[trans-4-(4-Butylphenyl)cyclohexyl]-2-(3-chlor-4-butylphenyl)-acetylen
1-[trans-4-(4-Pentylphenyl)cyclohexyl]-2-(3-chlor-4-butylphenyl)-acetylen
1-[trans-4-(4-Hexylphenyl)cyclohexyl]-2-(3-chlor-4-butylphenyl)-acetylen
1-[trans-4-(4-Heptylphenyl)cyclohexyl]-2-(3-chlor-4-butylphenyl)-acetylen
1-[trans-4-(4-Methylphenyl)cyclohexyl]-2-(3-cyan-4-butylphenyl)-acetylen
1-[trans-4-(4-Ethylphenyl)cyclohexyl]-2-(3-cyan-4-butylphenyl)-acetylen
1-[trans-4-(4-Propylphenyl)cyclohexyl]-2-(3-cyan-4-butylphenyl)-acetylen
1-[trans-4-(4-Butylphenyl)cyclohexyl]-2-(3-cyan-4-butylphenyl)-acetylen
1-[trans-4-(4-Pentylphenyl)cyclohexyl]-2-(3-cyan-4-butylphenyl)-acetylen
1-[trans-4-(4-Hexylphenyl)cyclohexyl]-2-(3-cyan-4-butylphenyl)-acetylen
1-[trans-4-(4-Heptylphenyl)cyclohexyl]-2-(3-cyan-4-butylphenyl)-acetylen
1-[trans-4-(4-Methylphenyl)cyclohexyl]-2-(2-fluor-4-pentylphenyl)-acetylen
1-[trans-4-(4-Ethylphenyl)cyclohexyl]-2-(2-fluor-4-pentylphenyl)-acetylen
1-[trans-4-(4-Propylphenyl)cyclohexyl]-2-(2-fluor-4-pentylphenyl)-acetylen
1-[trans-4-(4-Butylphenyl)cyclohexyl]-2-(2-fluor-4-pentylphenyl)-acetylen

1-[trans-4-(4-Pentylphenyl)cyclohexyl]-2-(2-fluor-4-pentylphenyl)-acetylen
1-[trans-4-(4-Hexylphenyl)cyclohexyl]-2-(2-fluor-4-pentylphenyl)-acetylen
1-[trans-4-(4-Heptylphenyl)cyclohexyl]-2-(2-fluor-4-pentylphenyl)-acetylen
1-[trans-4-(4-Methylphenyl)cyclohexyl]-2-(3-chlor-4-hexylphenyl)-acetylen
1-[trans-4-(4-Ethylphenyl)cyclohexyl]-2-(3-chlor-4-hexylphenyl)-acetylen
1-[trans-4-(4-Propylphenyl)cyclohexyl]-2-(3-chlor-4-hexylphenyl)-acetylen
1-[trans-4-(4-Butylphenyl)cyclohexyl]-2-(3-chlor-4-hexylphenyl)-acetylen
1-[trans-4-(4-Pentylphenyl)cyclohexyl]-2-(3-chlor-4-hexylphenyl)-acetylen
1-[trans-4-(4-Hexylphenyl)cyclohexyl]-2-(3-chlor-4-hexylphenyl)-acetylen
1-[trans-4-(4-Heptylphenyl)cyclohexyl]-2-(3-chlor-4-hexylphenyl)-acetylen
1-[trans-4-(4-Methylphenyl)cyclohexyl]-2-(2-cyan-4-heptylphenyl)-acetylen
1-[trans-4-(4-Ethylphenyl)cyclohexyl]-2-(2-cyan-4-heptylphenyl)-acetylen
1-[trans-4-(4-Propylphenyl)cyclohexyl]-2-(2-cyan-4-heptylphenyl)-acetylen
1-[trans-4-(4-Butylphenyl)cyclohexyl]-2-(2-cyan-4-heptylphenyl)-acetylen
1-[trans-4-(4-Pentylphenyl)cyclohexyl]-2-(2-cyan-4-heptylphenyl)-acetylen
1-[trans-4-(4-Hexylphenyl)cyclohexyl]-2-(2-cyan-4-heptylphenyl)-acetylen
1-[trans-4-(4-Heptylphenyl)cyclohexyl]-2-(2-cyan-4-heptylphenyl)-acetylen
1-[trans-4-(4-Methyloxyphenyl)cyclohexyl]-2-(4-propylphenyl)-acetylen
1-[trans-4-(4-Ethyloxyphenyl)cyclohexyl]-2-(4-propylphenyl)-acetylen
1-[trans-4-(4-Propyloxyphenyl)cyclohexyl]-2-(4-propylphenyl)-acetylen
1-[trans-4-(4-Butyloxyphenyl)cyclohexyl]-2-(4-propylphenyl)-acetylen
1-[trans-4-(4-Pentyloxyphenyl)cyclohexyl]-2-(4-propylphenyl)-acetylen
1-[trans-4-(4-Hexyloxyphenyl)cyclohexyl]-2-(4-propylphenyl)-acetylen
1-[trans-4-(4-Heptyloxyphenyl)cyclohexyl]-2-(4-propylphenyl)-acetylen
1-[trans-4-(4-Octyloxyphenyl)cyclohexyl]-2-(4-propylphenyl)-acetylen
1-[trans-4-(4-Nonyloxyphenyl)cyclohexyl]-2-(4-propylphenyl)-acetylen
1-[trans-4-(4-Decyloxyphenyl)cyclohexyl]-2-(4-propylphenyl)-acetylen
1-[trans-4-(4-Methyloxyphenyl)cyclohexyl]-2-(4-butylphenyl)-acetylen
1-[trans-4-(4-Ethyloxyphenyl)cyclohexyl]-2-(4-butylphenyl)-acetylen
1-[trans-4-(4-Propyloxyphenyl)cyclohexyl]-2-(4-butylphenyl)-acetylen
1-[trans-4-(4-Butyloxyphenyl)cyclohexyl]-2-(4-butylphenyl)-acetylen
1-[trans-4-(4-Pentyloxyphenyl)cyclohexyl]-2-(4-butylphenyl)-acetylen
1-[trans-4-(4-Hexyloxyphenyl)cyclohexyl]-2-(4-butylphenyl)-acetylen
1-[trans-4-(4-Heptyloxyphenyl)cyclohexyl]-2-(4-butylphenyl)-acetylen
1-[trans-4-(4-Methyloxyphenyl)cyclohexyl]-2-(4-pentylphenyl)-acetylen
1-[trans-4-(4-Ethyloxyphenyl)cyclohexyl]-2-(4-pentylphenyl)-acetylen
1-[trans-4-(4-Propyloxyphenyl)cyclohexyl]-2-(4-pentylphenyl)-acetylen
1-[trans-4-(4-Butyloxyphenyl)cyclohexyl]-2-(4-pentylphenyl)-acetylen
1-[trans-4-(4-Pentyloxyphenyl)cyclohexyl]-2-(4-pentylphenyl)-acetylen
1-[trans-4-(4-Hexyloxyphenyl)cyclohexyl]-2-(4-pentylphenyl)-acetylen
1-[trans-4-(4-Heptyloxyphenyl)cyclohexyl]-2-(4-pentylphenyl)-acetylen
1-[trans-4-(4-Methyloxyphenyl)cyclohexyl]-2-(4-hexylphenyl)-acetylen
1-[trans-4-(4-Ethyloxyphenyl)cyclohexyl]-2-(4-hexylphenyl)-acetylen
1-[trans-4-(4-Propyloxyphenyl)cyclohexyl]-2-(4-hexylphenyl)-acetylen
1-[trans-4-(4-Butyloxyphenyl)cyclohexyl]-2-(4-hexylphenyl)-acetylen
1-[trans-4-(4-Pentyloxyphenyl)cyclohexyl]-2-(4-hexylphenyl)-acetylen
1-[trans-4-(4-Hexyloxyphenyl)cyclohexyl]-2-(4-hexylphenyl)-acetylen
1-[trans-4-(trans-4-Methylcyclohexyl)cyclohexyl]-2-(4-propylphenyl)-acetylen
1-[trans-4-(trans-4-Ethylcyclohexyl)cyclohexyl]-2-(4-propylphenyl)-acetylen
1-[trans-4-(trans-4-Propylcyclohexyl)cyclohexyl]-2-(4-propylphenyl)-acetylen
1-[trans-4-(trans-4-Butylcyclohexyl)cyclohexyl]-2-(4-propylphenyl)-acetylen
1-[trans-4-(trans-4-Pentylcyclohexyl)cyclohexyl]-2-(4-propylphenyl)-acetylen
1-[trans-4-(trans-4-Hexylcyclohexyl)cyclohexyl]-2-(4-propylphenyl)-acetylen
1-[trans-4-(trans-4-Heptylcyclohexyl)cyclohexyl]-2-(4-propylphenyl)-acetylen
1-[trans-4-(trans-4-Methylcyclohexyl)cyclohexyl]-2-(4-butylphenyl)-acetylen
1-[trans-4-(trans-4-Ethylcyclohexyl)cyclohexyl]-2-(4-butylphenyl)-acetylen
1-[trans-4-(trans-4-Propylcyclohexyl)cyclohexyl]-2-(4-butylphenyl)-acetylen
1-[trans-4-(trans-4-Butylcyclohexyl)cyclohexyl]-2-(4-butylphenyl)-acetylen

1-[trans-4-(trans-4-Pentylcyclohexyl)cyclohexyl]-2-(4-butylphenyl)-acetylen
1-[trans-4-(trans-4-Hexylcyclohexyl)cyclohexyl]-2-(4-butylphenyl)-acetylen
1-[trans-4-(trans-4-Heptylcyclohexyl)cyclohexyl]-2-(4-butylphenyl)-acetylen
1-[trans-4-(trans-4-Methylcyclohexyl)cyclohexyl]-2-(4-pentylphenyl)-acetylen
1-[trans-4-(trans-4-Ethylcyclohexyl)cyclohexyl]-2-(4-pentylphenyl)-acetylen
1-[trans-4-(trans-4-Propylcyclohexyl)cyclohexyl]-2-(4-pentylphenyl)-acetylen
1-[trans-4-(trans-4-Butylcyclohexyl)cyclohexyl]-2-(4-pentylphenyl)-acetylen
1-[trans-4-(trans-4-Pentylcyclohexyl)cyclohexyl]-2-(4-pentylphenyl)-acetylen
1-[trans-4-(trans-4-Hexylcyclohexyl)cyclohexyl]-2-(4-pentylphenyl)-acetylen
1-[trans-4-(trans-4-Heptylcyclohexyl)cyclohexyl]-2-(4-pentylphenyl)-acetylen
1-[trans-4-(trans-4-Methylcyclohexyl)cyclohexyl]-2-(4-hexylphenyl)-acetylen
1-[trans-4-(trans-4-Ethylcyclohexyl)cyclohexyl]-2-(4-hexylphenyl)-acetylen
1-[trans-4-(trans-4-Propylcyclohexyl)cyclohexyl]-2-(4-hexylphenyl)-acetylen
1-[trans-4-(trans-4-Butylcyclohexyl)cyclohexyl]-2-(4-hexylphenyl)-acetylen
1-[trans-4-(trans-4-Pentylcyclohexyl)cyclohexyl]-2-(4-hexylphenyl)-acetylen
1-[trans-4-(trans-4-Hexylcyclohexyl)cyclohexyl]-2-(4-hexylphenyl)-acetylen
1-[trans-4-(trans-4-Heptylcyclohexyl)cyclohexyl]-2-(4-hexylphenyl)-acetylen
1-[trans-4-(trans-4-Methylcyclohexyl)cyclohexyl]-2-(3-fluor-4-ethylphenyl-acetylen
1-[trans-4-(trans-4-Ethylcyclohexyl)cyclohexyl]-2-(3-fluor-4-ethylphenyl-acetylen
1-[trans-4-(trans-4-Propylcyclohexyl)cyclohexyl]-2-(3-fluor-4-ethylphenyl-acetylen
1-[trans-4-(trans-4-Butylcyclohexyl)cyclohexyl]-2-(3-fluor-4-ethylphenyl)-acetylen
1-[trans-4-(trans-4-Pentylcyclohexyl)cyclohexyl]-2-(3-fluor-4-ethylphenyl)-acetylen
1-[trans-4-(trans-4-Hexylcyclohexyl)cyclohexyl]-2-(3-fluor-4-ethylphenyl)-acetylen
1-[trans-4-(trans-4-Heptylcyclohexyl)cyclohexyl]-2-(3-fluor-4-ethylphenyl)-acetylen
1-[trans-4-(trans-4-Methylcyclohexyl)cyclohexyl]-2-(3-fluor-4-propylphenyl)-acetylen
1-[trans-4-(trans-4-Ethylcyclohexyl)cyclohexyl]-2-(3-fluor-4-propylphenyl)-acetylen
1-[trans-4-(trans-4-Propylcyclohexyl)cyclohexyl]-2-(3-fluor-4-propylphenyl)-acetylen
1-[trans-4-(trans-4-Butylcyclohexyl)cyclohexyl]-2-(3-fluor-4-propylphenyl)-acetylen
1-[trans-4-(trans-4-Pentylcyclohexyl)cyclohexyl]-2-(3-fluor-4-propylphenyl)-acetylen
1-[trans-4-(trans-4-Hexylcyclohexyl)cyclohexyl]-2-(3-fluor-4-propylphenyl)-acetylen
1-[trans-4-(trans-4-Heptylcyclohexyl)cyclohexyl]-2-(3-fluor-4-propylphenyl)-acetylen
1-[trans-4-(trans-4-Methylcyclohexyl)cyclohexyl]-2-(3-fluor-4-butylphenyl)-acetylen
1-[trans-4-(trans-4-Ethylcyclohexyl)cyclohexyl]-2-(3-fluor-4-butylphenyl)-acetylen
1-[trans-4-(trans-4-Propylcyclohexyl)cyclohexyl]-2-(3-fluor-4-butylphenyl)-acetylen
1-[trans-4-(trans-4-Butylcyclohexyl)cyclohexyl]-2-(3-fluor-4-butylphenyl)-acetylen
1-[trans-4-(trans-4-Pentylcyclohexyl)cyclohexyl]-2-(3-fluor-4-butylphenyl)-acetylen
1-[trans-4-(trans-4-Hexylcyclohexyl)cyclohexyl]-2-(3-fluor-4-butylphenyl)-acetylen
1-[trans-4-(trans-4-Heptylcyclohexyl)cyclohexyl]-2-(3-fluor-4-butylphenyl)-acetylen
1-[trans-4-(trans-4-Methylcyclohexyl)cyclohexyl]-2-(3-fluor-4-pentylphenyl)-acetylen
1-[trans-4-(trans-4-Ethylcyclohexyl)cyclohexyl]-2-(3-fluor-4-pentylphenyl)-acetylen
1-[trans-4-(trans-4-Propylcyclohexyl)cyclohexyl]-2-(3-fluor-4-pentylphenyl)-acetylen
1-[trans-4-(trans-4-Butylcyclohexyl)cyclohexyl]-2-(3-fluor-4-pentylphenyl)-acetylen
1-[trans-4-(trans-4-Pentylcyclohexyl)cyclohexyl]-2-(3-fluor-4-pentylphenyl)-acetylen
1-[trans-4-(trans-4-Hexylcyclohexyl)cyclohexyl]-2-(3-fluor-4-pentylphenyl)-acetylen
1-[trans-4-(trans-4-Heptylcyclohexyl)cyclohexyl]-2-(3-fluor-4-pentylphenyl)-acetylen
1-[trans-4-(trans-4-Methylcyclohexyl)cyclohexyl]-2-(3-chlor-4-pentylphenyl)-acetylen
1-[trans-4-(trans-4-Ethylcyclohexyl)cyclohexyl]-2-(3-chlor-4-pentylphenyl)-acetylen
1-[trans-4-(trans-4-Propylcyclohexyl)cyclohexyl]-2-(3-chlor-4-pentylphenyl )-acetylen
1-[trans-4-(trans-4-Butylcyclohexyl)cyclohexyl]-2-(3-chlor-4-pentylphenyl)-acetylen
1-[trans-4-(trans-4-Pentylcyclohexyl)cyclohexyl]-2-(3-chlor-4-pentylphenyl)-acetylen
1-[trans-4-(trans-4-Hexylcyclohexyl)cyclohexyl]-2-(3-chlor-4-pentylphenyl)-acetylen
1-[trans-4-(trans-4-Heptylcyclohexyl)cyclohexyl]-2-(3-chlor-4-pentylphenyl)-acetylen
1-[trans-4-(trans-4-Methylcyclohexyl)cyclohexyl]-2-(3,4-difluorphenyl)-acetylen
1-[trans-4-(trans-4-Ethylcyclohexyl)cyclohexyl]-2-(3,4-difluorphenyl)-acetylen
1-[trans-4-(trans-4-Propylcyclohexyl)cyclohexyl]-2-(3,4-difluorphenyl)-acetylen
1-[trans-4-(trans-4-Butylcyclohexyl)cyclohexyl]-2-(3,4-difluorphenyl)-acetylen
1-[trans-4-(trans-4-Pentylcyclohexyl)cyclohexyl]-2-(3,4-difluorphenyl)-acetylen
1-[trans-4-(trans-4-Hexylcyclohexyl)cyclohexyl]-2-(3,4-difluorphenyl)-acetylen

1-[trans-4-(trans-4-Heptylcyclohexyl)cyclohexyl]-2-(3,4-difluorphenyl)-acetylen
1-[trans-4-(trans-4-Methylcyclohexyl)cyclohexyl]-2-(4-cyanphenyl)-acetylen
1-[trans-4-(trans-4-Ethylcyclohexyl)cyclohexyl]-2-(4-cyanphenyl)-acetylen
1-[trans-4-(trans-4-Propylcyclohexyl)cyclohexyl]-2-(4-cyanphenyl)-acetylen
1-[trans-4-(trans-4-Butylcyclohexyl)cyclohexyl]-2-(4-cyanphenyl)-acetylen
1-[trans-4-(trans-4-Pentylcyclohexyl)cyclohexyl]-2-(4-cyanphenyl)-acetylen
1-[trans-4-(trans-4-Hexylcyclohexyl)cyclohexyl]-2-(4-cyanphenyl)-acetylen
1-[trans-4-(trans-4-Heptylcyclohexyl)cyclohexyl]-2-(4-cyanphenyl)-acetylen
1-[trans-4-(trans-4-Methylcyclohexyl)cyclohexyl]-2-(2-fluor-4-pentylcyclohexyl)-acetylen
1-[trans-4-(trans-4-Ethylcyclohexyl)cyclohexyl]-2-(2-fluor-4-pentylcyclohexyl)-acetylen
1-[trans-4-(trans-4-Propylcyclohexyl)cyclohexyl]-2-(2-fluor-4-pentylcyclohexyl)-acetylen
1-[trans-4-(trans-4-Butylcyclohexyl)cyclohexyl]-2-(2-fluor-4-pentylcyclohexyl)-acetylen
1-[trans-4-(trans-4-Pentylcyclohexyl)cyclohexyl]-2-(2-fluor-4-pentylcyclohexyl)-acetylen
1-[trans-4-(trans-4-Hexylcyclohexyl)cyclohexyl]-2-(2-fluor-4-pentylcyclohexyl)-acetylen
1-[trans-4-(trans-4-Heptylcyclohexyl)cyclohexyl]-2-(2-fluor-4-pentylcyclohexyl)-acetylen
1-[trans-4-(trans-4-Methylcyclohexyl)cyclohexyl]-2-(4-propyloxyphenyl)-acetylen
1-[trans-4-(trans-4-Ethylcyclohexyl)cyclohexyl]-2-(4-propyloxyphenyl)-acetylen
1-[trans-4-(trans-4-Propylcyclohexyl)cyclohexyl]-2-(4-propylocyphenyl)-acetylen
1-[trans-4-(trans-4-Butylcyclohexyl)cyclohexyl]-2-(4-propyloxyphenyl)-acetylen
1-[trans-4-(trans-4-Pentylcyclohexyl)cyclohexyl]-2-(4-propyloxyphenyl)-acetylen
1-[trans-4-(trans-4-Hexylcyclohexyl)cyclohexyl]-2-(4-propyloxyphenyl)-acetylen
1-[trans-4-(trans-4-Heptylcyclohexyl)cyclohexyl]-2-(4-propyloxyphenyl)-acetylen
1-[trans-4-(trans-4-Methylcyclohexyl)cyclohexyl]-2-(4-butyloxyphenyl)-acetylen
1-[trans-4-(trans-4-Ethylcyclohexyl)cyclohexyl]-2-(4-butyloxyphenyl)-acetylen
1-[trans-4-(trans-4-Propylcyclohexyl)cyclohexyl]-2-(4-butyloxyphenyl)-acetylen
1-[trans-4-(trans-4-Butylcyclohexyl)cyclohexyl]-2-(4-butyloxyohenyl)-acetylen
1-[trans-4-(trans-4-Pentylcyclohexyl)cyclohexyl]-2-(4-butyloxyphenyl)-acetylen
1-[trans-4-(trans-4-Hexylcyclohexyl)cyclohexyl]-2-(4-butyloxyphenyl)-acetylen
1-[trans-4-(trans-4-Heptylcyclohexyl)cyclohexyl]-2-(4-butyloxyphenyl)-acetylen
1-[trans-4-(trans-4-Methylcyclohexyl)cyclohexyl]-2-(4-pentyloxyphenyl)-acetylen
1-[trans-4-(trans-4-Ethylcyclohexyl)cyclohexyl]-2-(4-pentyloxyphenyl)-acetylen
1-[trans-4-(trans-4-Propylcyclohexyl)cyclohexyl]-2-(4-pentyloxyphenyl)-acetylen
1-[trans-4-(trans-4-Butylcyclohexyl)cyclohexyl]-2-(4-pentyloxyphenyl)-acetylen
1-[trans-4-(trans-4-Pentylcyclohexyl)cyclohexyl]-2-(4-pentyloxyphenyl)-acetylen
1-[trans-4-(trans-4-Hexylcyclohexyl)cyclohexyl]-2-(4-pentyloxyphenyl)-acetylen
1-[trans-4-(trans-4-Heptylcyclohexyl)cyclohexyl]-2-(4-pentyloxyphenyl)-acetylen
1-(trans-4-Methylcyclohexyl)-2-(4'-propylbiphenyl-4-yl)-acetylen
1-(trans-4-Ethylcyclohexyl)-2-(4'-propylbiphenyl-4-yl)-acetylen
1-(trans-4-Propylcyclohexyl)-2-(4'-propylbiphenyl-4-yl)-acetylen
1-(trans-4-Butylcyclohexyl)-2-(4'-propylbiphenyl-4-yl)-acetylen
1-(trans-4-Pentylcyclohexyl)-2-(4'-propylbiphenyl-4-yl)-acetylen
1-(trans-4-Hexylcyclohexyl)-2-(4'-propylbiphenyl-4-yl)-acetylen
1-(trans-4-Heptylcyclohexyl)-2-(4'-propylbiphenyl-4-yl)-acetylen
1-(trans-4-Octylcyclohexyl)-2-(4'-propylbiphenyl-4-yl)-acetylen
1-(trans-4-Methylcyclohexyl)-2-(4'-butylbiphenyl-4-yl)-acetylen
1-(trans-4-Ethylcyclohexyl)-2-(4'-butylbiphenyl-4-yl)-acetylen
1-(trans-4-Propylcyclohexyl)-2-(4'-butylbiphenyl-4-yl)-acetylen
1-(trans-4-Butylcyclohexyl)-2-(4'-butylbiphenyl-4-yl)-acetylen
1-(trans-4-Pentylcyclohexyl)-2-(4'-butylbiphenyl-4-yl)-acetylen
1-(trans-4-Hexylcyclohexyl)-2-(4'-butylbiphenyl-4-yl)-acetylen
1-(trans-4-Heptylcyclohexyl)-2-(4'-butylbiphenyl-4-yl)-acetylen
1-(trans-4-Methylcyclohexyl)-2-(4'-pentylbiphenyl-4-yl)-acetylen
1-(trans-4-Ethylcyclohexyl)-2-(4'-pentylbiphenyl-4-yl)-acetylen
1-(trans-4-Propylcyclohexyl)-2-(4'-pentylbiphenyl-4-yl)-acetylen
1-(trans-4-Butylcyclohexyl)-2-(4'-pentylbiphenyl-4-yl)-acetylen
1-(trans-4-Pentylcyclohexyl)-2-(4'-pentylbiphenyl-4-yl)-acetylen
1-(trans-4-Hexylcyclohexyl)-2-(4'-pentylbiphenyl-4-yl)-acetylen
1-(trans-4-Heptylcyclohexyl)-2-(4'-pentylbiphenyl-4-yl)-acetylen

1-(trans-4-Methylcyclohexyl)-2-(4'-hexylbiphenyl-4-yl)-acetylen
1-(trans-4-Ethylcyclohexyl)-2-(4'-hexylbiphenyl-4-yl)-acetylen
1-(trans-4-Propylcyclohexyl)-2-(4'-hexylbiphenyl-4-yl)-acetylen
1-(trans-4-Butylcyclohexyl)-2-(4'-hexylbiphenyl-4-yl)-acetylen
1-(trans-4-Pentylcyclohexyl)-2-(4'-hexylbiphenyl-4-yl)-acetylen
1-(trans-4-Hexylcyclohexyl)-2-(4'-hexylbiphenyl-4-yl)-acetylen
1-(trans-4-Heptylcyclohexyl)-2-(4'-hexylbiphenyl-4-yl)-acetylen
1-(trans-4-Methylcyclohexyl)-2-(4'-heptylbiphenyl-4-yl)-acetylen
1-(trans-4-Ethylcyclohexyl)-2-(4'-heptylbiphenyl-4-yl)-acetylen
1-(trans-4-Propylcyclohexyl)-2-(4'-heptylbiphenyl-4-yl)-acetylen
1-(trans-4-Butylcyclohexyl)-2-(4'-heptylbiphenyl-4-yl)-acetylen
1-(trans-4-Pentylcyclohexyl)-2-(4'-heptylbiphenyl-4-yl)-acetylen
1-(trans-4-Hexylcyclohexyl)-2-(4'-heptylbiphenyl-4-yl)-acetylen
1-(trans-4-Heptylcyclohexyl)-2-(4'-heptylbiphenyl-4-yl)-acetylen
1-(trans-4-Methylcyclohexyl)-2-(4'-ethoxybiphenyl-4-yl)-acetylen
1-(trans-4-Ethylcyclohexyl)-2-(4'-ethoxybiphenyl-4-yl)-acetylen
1-(trans-4-Propylcyclohexyl)-2-(4'-ethoxybiphenyl-4-yl)-acetylen
1-(trans-4-Butylcyclohexyl)-2-(4'-ethoxybiphenyl-4-yl)-acetylen
1-(trans-4-Pentylcyclohexyl)-2-(4'-ethoxybiphenyl-4-yl)-acetylen
1-(trans-4-Hexylcyclohexyl)-2-(4'-ethoxybiphenyl-4-yl)-acetylen
1-(trans-4-Heptylcyclohexyl)-2-(4'-ethoxybiphenyl-4-yl)-acetylen
1-(trans-4-Octylcyclohexyl)-2-(4'-ethoxybiphenyl-4-yl)-acetylen
1-(trans-4-Nonylcyclohexyl)-2-(4'-ethoxybiphenyl-4-yl)-acetylen
1-(trans-4-Decylcyclohexyl)-2-(4'-ethoxybiphenyl-4-yl)-acetylen
1-(trans-4-Methylcyclohexyl)-2-(4'-propyloxybiphenyl-4-yl)-acetylen
1-(trans-4-Ethylcyclohexyl)-2-(4'-propyloxybiphenyl-4-yl)-acetylen
1-(trans-4-Propylcyclohexyl)-2-(4'-propyloxybiphenyl-4-yl)-acetylen
1-(trans-4-Butylcyclohexyl)-2-(4'-propyloxybiphenyl-4-yl)-acetylen
1-(trans-4-Pentylcyclohexyl)-2-(4'-propyloxybiphenyl-4-yl)-acetylen
1-(trans-4-Hexylcyclohexyl)-2-(4'-propyloxybiphenyl-4-yl)-acetylen
1-(trans-4-Heptylcyclohexyl)-2-(4'-propyloxybiphenyl-4-yl)-acetylen
1-(trans-4-Octylcyclohexyl)-2-(4'-propyloxybiphenyl-4-yl)-acetylen
1-(trans-4-Methylcyclohexyl)-2-(4'-butyloxybiphenyl-4-yl)-acetylen
1-(trans-4-Ethylcyclohexyl)-2-(4'-butyloxybiphenyl-4-yl)-acetylen
1-(trans-4-Propylcyclohexyl)-2-(4'-butyloxybiphenyl-4-yl)-acetylen
1-(trans-4-Butylcyclohexyl)-2-(4'-butyloxybiphenyl-4-yl)-acetylen
1-(trans-4-Pentylcyclohexyl)-2-(4'-butyloxybiphenyl-4-yl)-acetylen
1-(trans-4-Hexylcyclohexyl)-2-(4'-butyloxybiphenyl-4-yl)-acetylen
1-(trans-4-Heptylcyclohexyl)-2-(4'-butyloxybiphenyl-4-yl)-acetylen
1-(trans-4-Methylcyclohexyl)-2-(4'-pentyloxybiphenyl-4-yl)-acetylen
1-(trans-4-Ethylcyclohexyl)-2-(4'-pentyloxybiphenyl-4-yl)-acetylen
1-(trans-4-Propylcyclohexyl)-2-(4'-pentyloxybiphenyl-4-yl)-acetylen
1-(trans-4-Butylcyclohexyl)-2-(4'-pentyloxybiphenyl-4-yl)-acetylen
1-(trans-4-Pentylcyclohexyl)-2-(4'-pentyloxybiphenyl-4-yl)-acetylen
1-(trans-4-Hexylcyclohexyl)-2-(4'-pentyloxybiphenyl-4-yl)-acetylen
1-(trans-4-Heptylcyclohexyl)-2-(4'-pentyloxybiphenyl-4-yl)-acetylen
1-(trans-4-Methylcyclohexyl)-2-(4'-hexyloxybiphenyl-4-yl)-acetylen
1-(trans-4-Ethylcyclohexyl)-2-(4'-hexyloxybiphenyl-4-yl)-acetylen
1-(trans-4-Propylcyclohexyl)-2-(4'-hexyloxybiphenyl-4-yl)-acetylen
1-(trans-4-Butylcyclohexyl)-2-(4'-hexyloxybiphenyl-4-yl)-acetylen
1-(trans-4-Pentylcyclohexyl)-2-(4'-hexyloxybiphenyl-4-yl)-acetylen
1-(trans-4-Hexylcyclohexyl)-2-(4'-hexyloxybiphenyl-4-yl)-acetylen
1-(trans-4-Heptylcyclohexyl)-2-(4'-hexyloxybiphenyl-4-yl)-acetylen
1-(trans-4-Methylcyclohexyl)-2-(4'-heptyloxybiphenyl-4-yl)-acetylen
1-(trans-4-Ethylcyclohexyl)-2-(4'-heptyloxybiphenyl-4-yl)-acetylen
1-(trans-4-Propylcyclohexyl)-2-(4'-heptyloxybiphenyl-4-yl)-acetylen
1-(trans-4-Butylcyclohexyl)-2-(4'-heptyloxybiphenyl-4-yl)-acetylen
1-(trans-4-Pentylcyclohexyl)-2-(4'-heptyloxybiphenyl-4-yl)-acetylen

1-(trans-4-Hexylcyclohexyl)-2-(4'-heptyloxybiphenyl-4-yl)-acetylen
1-(trans-4-Methylcyclohexyl)-2-(3'-fluor-4'-propylbiphenyl-4-yl)-acetylen
1-(trans-4-Ethylcyclohexyl)-2-(3'-fluor-4'-propylbiphenyl-4-yl)-acetylen
1-(trans-4-Propylcyclohexyl)-2-(3'-fluor-4'-propylbiphenyl-4-yl)-acetylen
1-(trans-4-Butylcyclohexyl)-2-(3'-fluor-4'-propylbiphenyl-4-yl)-acetylen
1-(trans-4-Pentylcyclohexyl)-2-(3'-fluor-4'-propylbiphenyl-4-yl)-acetylen
1-(trans-4-Hexylcyclohexyl)-2-(3'-fluor-4'-propylbiphenyl-4-yl)-acetylen
1-(trans-4-Heptylcyclohexyl)-2-(3'-fluor-4'-propylbiphenyl-4-yl)-acetylen
1-(trans-4-Methylcyclohexyl)-2-(3'-fluor-4'-butylbiphenyl-4-yl)-acetylen
1-(trans-4-Ethylcyclohexyl)-2-(3'-fluor-4'-butylbiphenyl-4-yl)-acetylen
1-(trans-4-Propylcyclohexyl)-2-(3'-fluor-4'-butylbiphenyl-4-yl)-acetylen
1-(trans-4-Butylcyclohexyl)-2-(3'-fluor-4'-butylbiphenyl-4-yl)-acetylen
1-(trans-4-Pentylcyclohexyl)-2-(3'-fluor-4'-butylbiphenyl-4-yl)-acetylen
1-(trans-4-Hexylcyclohexyl)-2-(3'-fluor-4'-butylbiphenyl-4-yl)-acetylen
1-(trans-4-Heptylcyclohexyl)-2-(3'-fluor-4'-butylbiphenyl-4-yl)-acetylen
1-(trans-4-Octylcyclohexyl)-2-(3'-fluor-4'-butylbiphenyl-4-yl)-acetylen
1-(trans-4-Methylcyclohexyl)-2-(3'-fluor-4'-pentylbiphenyl-4-yl)-acetylen
1-(trans-4-Ethylcyclohexyl)-2-(3'-fluor-4'-pentylbiphenyl-4-yl)-acetylen
1-(trans-4-Propylcyclohexyl)-2-(3'-fluor-4'-pentylbiphenyl-4-yl)-acetylen
1-(trans-4-Butylcyclohexyl)-2-(3'-fluor-4'-pentylbiphenyl-4-yl)-acetylen
1-(trans-4-Pentylcyclohexyl)-2-(3'-fluor-4'-pentylbiphenyl-4-yl)-acetylen
1-(trans-4-Hexylcyclohexyl)-2-(3'-fluor-4'-pentylbiphenyl-4-yl)-acetylen
1-(trans-4-Heptylcyclohexyl)-2-(3'-fluor-4'-pentylbiphenyl-4-yl)-acetylen
1-(trans-4-Methylcyclohexyl)-2-(2-fluor-4'-butylbiphenyl-4-yl)-acetylen
1-(trans-4-Ethylcyclohexyl)-2-(2-fluor-4'-butylbiphenyl-4-yl)-acetylen
1-(trans-4-Propylcyclohexyl)-2-(2-fluor-4'-butylbiphenyl-4-yl)-acetylen
1-(trans-4-Butylcyclohexyl)-2-(2-fluor-4'-butylbiphenyl-4-yl)-acetylen
1-(trans-4-Pentylcyclohexyl)-2-(2-fluor-4'-butylbiphenyl-4-yl)-acetylen
1-(trans-4-Hexylcyclohexyl)-2-(2-fluor-4'-butylbiphenyl-4-yl)-acetylen
1-(trans-4-Heptylcyclohexyl)-2-(2-fluor-4'-butylbiphenyl-4-yl)-acetylen
1-(trans-4-Octylcyclohexyl)-2-(2-fluor-4'-butylbiphenyl-4-yl)-acetylen
1-(trans-4-Methylcyclohexyl)-2-(2-chlor-4'-pentylbiphenyl-4-yl)-acetylen
1-(trans-4-Ethylcyclohexyl)-2-(2-chlor-4'-pentylbiphenyl-4-yl)-acetylen
1-(trans-4-Propylcyclohexyl)-2-(2-chlor-4'-pentylbiphenyl-4-yl)-acetylen
1-(trans-4-Butylcyclohexyl)-2-(2-chlor-4'-pentylbiphenyl-4-yl)-acetylen
1-(trans-4-Pentylcyclohexyl)-2-(2-chlor-4'-pentylbiphenyl-4-yl)-acetylen
1-(trans-4-Hexylcyclohexyl)-2-(2-chlor-4'-pentylbiphenyl-4-yl)-acetylen
1-(trans-4-Heptylcyclohexyl)-2-(2-chlor-4'-pentylbiphenyl-4-yl)-acetylen
1-(trans-4-Methylcyclohexyl)-2-(4'-cyanbiphenyl-4-yl)-acetylen
1-(trans-4-Ethylcyclohexyl)-2-(4'-cyanbiphenyl-4-yl)-acetylen
1-(trans-4-Propylcyclohexyl)-2-(4'-cyanbiphenyl-4-yl)-acetylen
1-(trans-4-Butylcyclohexyl)-2-(4'-cyanbiphenyl-4-yl)-acetylen
1-(trans-4-Pentylcyclohexyl)-2-(4'-cyanbiphenyl-4-yl)-acetylen
1-(trans-4-Hexylcyclohexyl)-2-(4'-cyanbiphenyl-4-yl)-acetylen
1-(trans-4-Heptylcyclohexyl)-2-(4'-cyanbiphenyl-4-yl)-acetylen
1-(trans-4-Octylcyclohexyl)-2-(4'-cyanbiphenyl-4-yl)-acetylen
1-(trans-4-Nonylcyclohexyl)-2-(4'-cyanbiphenyl-4-yl)-acetylen
1-(trans-4-Decylcyclohexyl)-2-(4'-cyanbiphenyl-4-yl)-acetylen.

Beispiel 2

0,2 m 4-Propylbenzaldehyd und 0,2 mol 2-Methyl-5-methylpyridin werden zusammen mit 3 g Zinkchlorid 2 Tage auf 200° erhitzt. Der Verlauf der Reaktion kann mit Hilfe der Dünnschichtchromatographie verfolgt werden. Nach beendeter Reaktion wird überschüssiges Ausgangsmaterial abdestilliert und der Rückstand durch Kristallisation oder Chromatographie gereinigt.

0,1 mol des so erhaltenen Stilbenderivats werden in 200 ml Eisessig bei Raumtemperatur unter Rühren mit 0,1 mol $Br_2$ bromiert. Nach beendeter Zugabe erhitzt man kurz zum Sieden. Danach wird der Eisessig abgedampft und der Rückstand mit 200 ml tert.-Butanol versetzt. Zu diesem Gemisch gibt man bei

Raumtemperatur 0,4 mol Kalium-tert.-butanolat und erhitzt danach 3 Stunden zum Sieden. Nach Abkühlen wird Wasser zugesetzt und mit Ether extrahiert. Nach Aufarbeitung der organischen Phase und Reinigung durch Chromatographie erhält man 1-(4-Propylphenyl)-2-(5-methyl-pyridin-2-yl)-acetylen mit F. = 76°, K. = -20° (extr.) und $\Delta\epsilon$ = -1,2.

Analog werden hergestellt:

1-(4-Propylphenyl)-2-(5-ethyl-pyridin-2-yl)-acetylen
1-(4-Propylphenyl)-2-(5-propyl-pyridin-2-yl)-acetylen
1-(4-Propylphenyl)-2-(5-butyl-pyridin-2-yl)-acetylen
1-(4-Propylphenyl)-2-(5-pentyl-pyridin-2-yl)-acetylen
1-(4-Propylphenyl)-2-(5-hexyl-pyridin-2-yl)-acetylen
1-(4-Propylphenyl)-2-(5-heptyl-pyridin-2-yl)-acetylen
1-(4-Propylphenyl)-2-(5-octyl-pyridin-2-yl)-acetylen
1-(4-Ethylphenyl)-2-(5-methyl-pyridin-2-yl)-acetylen
1-(4-Ethylphenyl)-2-(5-ethyl-pyridin-2-yl)-acetylen
1-(4-Ethylphenyl)-2-(5-propyl-pyridin-2-yl)-acetylen
1-(4-Ethylphenyl)-2-(5-butyl-pyridin-2-yl)-acetylen
1-(4-Ethylphenyl)-2-(5-pentyl-pyridin-2-yl)-acetylen
1-(4-Ethylphenyl)-2-(5-hexyl-pyridin-2-yl)-acetylen
1-(4-Ethylphenyl)-2-(5-heptyl-pyridin-2-yl)-acetylen
1-(4-Ethylphenyl)-2-(5-octyl-pyridin-2-yl)-acetylen
1-(4-Butylphenyl)-2-(5-methyl-pyridin-2-yl)-acetylen
1-(4-Butylphenyl)-2-(5-ethyl-pyridin-2-yl)-acetylen
1-(4-Butylphenyl)-2-(5-propyl-pyridin-2-yl)-acetylen
1-(4-Butylphenyl)-2-(5-butyl-pyridin-2-yl)-acetylen
1-(4-Butylphenyl)-2-(5-pentyl-pyridin-2-yl)-acetylen
1-(4-Butylphenyl)-2-(5-hexyl-pyridin-2-yl)-acetylen
1-(4-Butylphenyl)-2-(5-heptyl-pyridin-2-yl)-acetylen
1-(4-Butylphenyl)-2-(5-octyl-pyridin-2-yl)-acetylen
1-(4-Pentylphenyl)-2-(5-methyl-pyridin-2-yl)-acetylen
1-(4-Pentylphenyl)-2-(5-ethyl-pyridin-2-yl)-acetylen
1-(4-Pentylphenyl)-2-(5-propyl-pyridin-2-yl)-acetylen
1-(4-Pentylphenyl)-2-(5-butyl-pyridin-2-yl)-acetylen
1-(4-Pentylphenyl)-2-(5-pentyl-pyridin-2-yl)-acetylen
1-(4-Pentylphenyl)-2-(5-hexyl-pyridin-2-yl)-acetylen
1-(4-Pentylphenyl)-2-(5-heptyl-pyridin-2-yl)-acetylen
1-(4-Pentylphenyl)-2-(5-octyl-pyridin-2-yl)-acetylen
1-(4-Hexylphenyl)-2-(5-methyl-pyridin-2-yl)-acetylen
1-(4-Hexylphenyl)-2-(5-ethyl-pyridin-2-yl)-acetylen
1-(4-Hexylphenyl)-2-(5-propyl-pyridin-2-yl)-acetylen
1-(4-Hexylphenyl)-2-(5-butyl-pyridin-2-yl)-acetylen
1-(4-Hexylphenyl)-2-(5-pentyl-pyridin-2-yl)-acetylen
1-(4-Hexylphenyl)-2-(5-hexyl-pyridin-2-yl)-acetylen
1-(4-Hexylphenyl)-2-(5-heptyl-pyridin-2-yl)-acetylen
1-(4-Hexylphenyl)-2-(5-octyl-pyridin-2-yl)-acetylen
1-(4-Heptylphenyl)-2-(5-methyl-pyridin-2-yl)-acetylen
1-(4-Heptylphenyl)-2-(5-ethyl-pyridin-2-yl)-acetylen
1-(4-Heptylphenyl)-2-(5-propyl-pyridin-2-yl)-acetylen
1-(4-Heptylphenyl)-2-(5-butyl-pyridin-2-yl)-acetylen
1-(4-Heptylphenyl)-2-(5-pentyl-pyridin-2-yl)-acetylen
1-(4-Heptylphenyl)-2-(5-hexyl-pyridin-2-yl)-acetylen
1-(4-Heptylphenyl)-2-(5-heptyl-pyridin-2-yl)-acetylen
1-(4-Heptylphenyl)-2-(5-octyl-pyridin-2-yl)-acetylen
1-(4-Octylphenyl)-2-(5-methyl-pyridin-2-yl)-acetylen
1-(4-Octylphenyl)-2-(5-ethyl-pyridin-2-yl)-acetylen
1-(4-Octylphenyl)-2-(5-propyl-pyridin-2-yl)-acetylen
1-(4-Octylphenyl)-2-(5-butyl-pyridin-2-yl)-acetylen
1-(4-Octylphenyl)-2-(5-pentyl-pyridin-2-yl)-acetylen
1-(4-Octylphenyl)-2-(5-hexyl-pyridin-2-yl)-acetylen

1-(4-Octylphenyl)-2-(5-heptyl-pyridin-2-yl)-acetylen
1-(4-Octylphenyl)-2-(5-octyl-pyridin-2-yl)-acetylen
1-(4-Ethylphenyl)-2-(5-methyloxy-pyridin-2-yl)-acetylen
1-(4-Ethylphenyl)-2-(5-ethyloxy-pyridin-2-yl)-acetylen
1-(4-Ethylphenyl)-2-(5-propyloxy-pyridin-2-yl)-acetylen
1-(4-Ethylphenyl)-2-(5-butyloxy-pyridin-2-yl)-acetylen
1-(4-Ethylphenyl)-2-(5-pentyloxy-pyridin-2-yl)-acetylen
1-(4-Ethylphenyl)-2-(5-hexyloxy-pyridin-2-yl)-acetylen
1-(4-Ethylphenyl)-2-(5-heptyloxy-pyridin-2-yl)-acetylen
1-(4-Ethylphenyl)-2-(5-octyloxy-pyridin-2-yl)-acetylen
1-(4-Propylphenyl)-2-(5-methyloxy-pyridin-2-yl)-acetylen
1-(4-Propylphenyl)-2-(5-ethyloxy-pyridin-2-yl)-acetylen
1-(4-Propylphenyl)-2-(5-propyloxy-pyridin-2-yl)-acetylen
1-(4-Propylphenyl)-2-(5-butyloxy-pyridin-2-yl)-acetylen
1-(4-Propylphenyl)-2-(5-pentyloxy-pyridin-2-yl)-acetylen
1-(4-Propylphenyl)-2-(5-hexyloxy-pyridin-2-yl)-acetylen
1-(4-Propylphenyl)-2-(5-heptyloxy-pyridin-2-yl)-acetylen
1-(4-Propylphenyl)-2-(5-octyl-pyridin-2-yl)-acetylen
1-(4-Butylphenyl)-2-(5-methyloxy-pyridin-2-yl)-acetylen
1-(4-Butylphenyl)-2-(5-ethyloxy-pyridin-2-yl)-acetylen
1-(4-Butylphenyl)-2-(5-propyloxy-pyridin-2-yl)-acetylen
1-(4-Butylphenyl)-2-(5-butyloxy-pyridin-2-yl)-acetylen
1-(4-Butylphenyl)-2-(5-pentyloxy-pyridin-2-yl)-acetylen
1-(4-Butylphenyl)-2-(5-hexyloxy-pyridin-2-yl)-acetylen
1-(4-Butylphenyl)-2-(5-heptyloxy-pyridin-2-yl)-acetylen
1-(4-Butylphenyl)-2-(5-octyloxy-pyridin-2-yl)-acetylen
1-(4-Pentylphenyl)-2-(5-methyloxy-pyridin-2-yl)-acetylen
1-(4-Pentylphenyl)-2-(5-ethyloxy-pyridin-2-yl)-acetylen
1-(4-Pentylphenyl)-2-(5-propyloxy-pyridin-2-yl)-acetylen
1-(4-Pentylphenyl)-2-(5-butyloxy-pyridin-2-yl)-acetylen
1-(4-Pentylphenyl)-2-(5-pentyloxy-pyridin-2-yl)-acetylen
1-(4-Pentylphenyl)-2-(5-hexyloxy-pyridin-2-yl)-acetylen
1-(4-Pentylphenyl)-2-(5-heptyloxy-pyridin-2-yl)-acetylen
1-(4-Pentylphenyl)-2-(5-octyloxy-pyridin-2-yl)-acetylen
1-(4-Hexylphenyl)-2-(5-methyloxy-pyridin-2-yl)-acetylen
1-(4-Hexylphenyl)-2-(5-ethyloxy-pyridin-2-yl)-acetylen
1-(4-Hexylphenyl)-2-(5-propyloxy-pyridin-2-yl)-acetylen
1-(4-Hexylphenyl)-2-(5-butyloxy-pyridin-2-yl)-acetylen
1-(4-Hexylphenyl)-2-(5-pentyloxy-pyridin-2-yl)-acetylen
1-(4-Hexylphenyl)-2-(5-hexyloxy-pyridin-2-yl)-acetylen
1-(4-Hexylphenyl)-2-(5-heptyloxy-pyridin-2-yl)-acetylen
1-(4-Hexylphenyl)-2-(5-octyloxy-pyridin-2-yl)-acetylen
1-(4-Heptylphenyl)-2-(5-methyloxy-pyridin-2-yl)-acetylen
1-(4-Heptylphenyl)-2-(5-ethyloxy-pyridin-2-yl)-acetylen
1-(4-Heptylphenyl)-2-(5-propyloxy-pyridin-2-yl)-acetylen
1-(4-Heptylphenyl)-2-(5-butyloxy-pyridin-2-yl)-acetylen
1-(4-Heptylphenyl)-2-(5-pentyloxy-pyridin-2-yl)-acetylen
1-(4-Heptylphenyl)-2-(5-hexyloxy-pyridin-2-yl)-acetylen
1-(4-Heptylphenyl)-2-(5-heptyloxy-pyridin-2-yl)-acetylen
1-(4-Heptylphenyl)-2-(5-octyloxy-pyridin-2-yl)-acetylen
1-(4-Octylphenyl)-2-(5-methyloxy-pyridin-2-yl)-acetylen
1-(4-Octylphenyl)-2-(5-ethyloxy-pyridin-2-yl)-acetylen
1-(4-Octylphenyl)-2-(5-propyloxy-pyridin-2-yl)-acetylen
1-(4-Octylphenyl)-2-(5-butyloxy-pyridin-2-yl)-acetylen
1-(4-Octylphenyl)-2-(5-pentyloxy-pyridin-2-yl)-acetylen
1-(4-Octylphenyl)-2-(5-hexyloxy-pyridin-2-yl)-acetylen
1-(4-Octylphenyl)-2-(5-heptyloxy-pyridin-2-yl)-acetylen
1-(4-Octylphenyl)-2-(5-octyloxy-pyridin-2-yl)-acetylen

1-(4-Ethyloxyphenyl)-2-(5-methyl-pyridin-2-yl)-acetylen
1-(4-Ethyloxyphenyl)-2-(5-ethyl-pyridin-2-yl)-acetylen
1-(4-Ethyloxyphenyl)-2-(5-propyl-pyridin-2-yl)-acetylen
1-(4-Ethyloxyphenyl)-2-(5-butyl-pyridin-2-yl)-acetylen
1-(4-Ethyloxyphenyl)-2-(5-pentyl-pyridin-2-yl)-acetylen
1-(4-Ethyloxyphenyl)-2-(5-hexyl-pyridin-2-yl)-acetylen
1-(4-Ethyloxyphenyl)-2-(5-heptyl-pyridin-2-yl)-acetylen
1-(4-Ethyloxyphenyl)-2-(5-octyl-pyridin-2-yl)-acetylen
1-(4-Propyloxyphenyl)-2-(5-methyl-pyridin-2-yl)-acetylen
1-(4-Propyloxyphenyl)-2-(5-ethyl-pyridin-2-yl)-acetylen
1-(4-Propyloxyphenyl)-2-(5-propyl-pyridin-2-yl)-acetylen
1-(4-Propyloxyphenyl)-2-(5-butyl-pyridin-2-yl)-acetylen
1-(4-Propyloxyphenyl)-2-(5-pentyl-pyridin-2-yl)-acetylen
1-(4-Propyloxyphenyl)-2-(5-hexyl-pyridin-2-yl)-acetylen
1-(4-Propyloxyphenyl)-2-(5-heptyl-pyridin-2-yl)-acetylen
1-(4-Propyloxyphenyl)-2-(5-octyl-pyridin-2-yl)-acetylen
1-(4-Butyloxyphenyl)-2-(5-methyl-pyridin-2-yl)-acetylen
1-(4-Butyloxyphenyl)-2-(5-ethyl-pyridin-2-yl)-acetylen
1-(4-Butyloxyphenyl)-2-(5-propyl-pyridin-2-yl)-acetylen
1-(4-Butyloxyphenyl)-2-(5-butyl-pyridin-2-yl)-acetylen
1-(4-Butyloxyphenyl)-2-(5-pentyl-pyridin-2-yl)-acetylen
1-(4-Butyloxyphenyl)-2-(5-hexyl-pyridin-2-yl)-acetylen
1-(4-Butyloxyphenyl)-2-(5-heptyl-pyridin-2-yl)-acetylen
1-(4-Butyloxyphenyl)-2-(5-octyl-pyridin-2-yl)-acetylen
1-(4-Pentyloxyphenyl)-2-(5-methyl-pyridin-2-yl)-acetylen
1-(4-Pentyloxyphenyl)-2-(5-ethyl-pyridin-2-yl)-acetylen
1-(4-Pentyloxyphenyl)-2-(5-propyl-pyridin-2-yl)-acetylen
1-(4-Pentyloxyphenyl)-2-(5-butyl-pyridin-2-yl)-acetylen
1-(4-Pentyloxyphenyl)-2-(5-pentyl-pyridin-2-yl)-acetylen
1-(4-Pentyloxyphenyl)-2-(5-hexyl-pyridin-2-yl)-acetylen
1-(4-Pentyloxyphenyl)-2-(5-heptyl-pyridin-2-yl)-acetylen
1-(4-Pentyloxyphenyl)-2-(5-octyl-pyridin-2-yl)-acetylen
1-(4-Hexyloxyphenyl)-2-(5-methyl-pyridin-2-yl)-acetylen
1-(4-Hexyloxyphenyl)-2-(5-ethyl-pyridin-2-yl)-acetylen
1-(4-Hexyloxyphenyl)-2-(5-propyl-pyridin-2-yl)-acetylen
1-(4-Hexyloxyphenyl)-2-(5-butyl-pyridin-2-yl)-acetylen
1-(4-Hexyloxyphenyl)-2-(5-pentyl-pyridin-2-yl)-acetylen
1-(4-Hexyloxyphenyl)-2-(5-hexyl-pyridin-2-yl)-acetylen
1-(4-Hexyloxyphenyl)-2-(5-heptyl-pyridin-2-yl)-acetylen
1-(4-Hexyloxyphenyl)-2-(5-octyl-pyridin-2-yl)-acetylen
1-(4-Heptyloxyphenyl)-2-(5-methyl-pyridin-2-yl)-acetylen
1-(4-Heptyloxyphenyl)-2-(5-ethyl-pyridin-2-yl)-acetylen
1-(4-Heptyloxyphenyl)-2-(5-propyl-pyridin-2-yl)-acetylen
1-(4-Heptyloxyphenyl)-2-(5-butyl-pyridin-2-yl)-acetylen
1-(4-Heptyloxyphenyl)-2-(5-pentyl-pyridin-2-yl)-acetylen
1-(4-Heptyloxyphenyl)-2-(5-hexyl-pyridin-2-yl)-acetylen
1-(4-Heptyloxyphenyl)-2-(5-heptyl-pyridin-2-yl)-acetylen
1-(4-Heptyloxyphenyl)-2-(5-octyl-pyridin-2-yl)-acetylen
1-(4-Octyloxyphenyl)-2-(5-methyl-pyridin-2-yl)-acetylen
1-(4-Octyloxyphenyl)-2-(5-ethyl-pyridin-2-yl)-acetylen
1-(4-Octyloxyphenyl)-2-(5-propyl-pyridin-2-yl)-acetylen
1-(4-Octyloxyphenyl)-2-(5-butyl-pyridin-2-yl)-acetylen
1-(4-Octyloxyphenyl)-2-(5-pentyl-pyridin-2-yl)-acetylen
1-(4-Octyloxyphenyl)-2-(5-hexyl-pyridin-2-yl)-acetylen
1-(4-Octyloxyphenyl)-2-(5-heptyl-pyridin-2-yl)-acetylen
1-(4-Octyloxyphenyl)-2-(5-octyl-pyridin-2-yl)-acetylen
1-(4-Propylphenyl)-2-(5-methyl-pyrimidin-2-yl)-acetylen
1-(4-Propylphenyl)-2-(5-ethyl-pyrimidin-2-yl)-acetylen

1-(4-Propylphenyl)-2-(5-propyl-pyrimidin-2-yl)-acetylen
1-(4-Propylphenyl)-2-(5-butyl-pyrimidin-2-yl)-acetylen
1-(4-Propylphenyl)-2-(5-pentyl-pyrimidin-2-yl)-acetylen
1-(4-Propylphenyl)-2-(5-hexyl-pyrimidin-2-yl)-acetylen
1-(4-Propylphenyl)-2-(5-heptyl-pyrimidin-2-yl)-acetylen
1-(4-Propylphenyl)-2-(5-octyl-pyrimidin-2-yl)-acetylen
1-(4-Butylphenyl)-2-(5-methyl-pyrimidin-2-yl)-acetylen
1-(4-Butylphenyl)-2-(5-ethyl-pyrimidin-2-yl)-acetylen
1-(4-Butylphenyl)-2-(5-propyl-pyrimidin-2-yl)-acetylen
1-(4-Butylphenyl)-2-(5-butyl-pyrimidin-2-yl)-acetylen
1-(4-Butylphenyl)-2-(5-pentyl-pyrimidin-2-yl)-acetylen
1-(4-Butylphenyl)-2-(5-hexyl-pyrimidin-2-yl)-acetylen
1-(4-Butylphenyl)-2-(5-heptyl-pyrimidin-2-yl)-acetylen
1-(4-Butylphenyl)-2-(5-octyl-pyrimidin-2-yl)-acetylen
1-(4-Pentylphenyl)-2-(5-methyl-pyrimidin-2-yl)-acetylen
1-(4-Pentylphenyl)-2-(5-ethyl-pyrimidin-2-yl)-acetylen
1-(4-Pentylphenyl)-2-(5-propyl-pyrimidin-2-yl)-acetylen
1-(4-Pentylphenyl)-2-(5-butyl-pyrimidin-2-yl)-acetylen
1-(4-Pentylphenyl)-2-(5-pentyl-pyrimidin-2-yl)-acetylen
1-(4-Pentylphenyl)-2-(5-hexyl-pyrimidin-2-yl)-acetylen
1-(4-Pentylphenyl)-2-(5-heptyl-pyrimidin-2-yl)-acetylen
1-(4-Pentylphenyl)-2-(5-octyl-pyrimidin-2-yl)-acetylen
1-(4-Hexylphenyl)-2-(5-methyl-pyrimidin-2-yl)-acetylen
1-(4-Hexylphenyl)-2-(5-ethyl-pyrimidin-2-yl)-acetylen
1-(4-Hexylphenyl)-2-(5-propyl-pyrimidin-2-yl)-acetylen
1-(4-Hexpylphenyl)-2-(5-butyl-pyrimidin-2-yl)-acetylen
1-(4-Hexylphenyl)-2-(5-pentyl-pyrimidin-2-yl)-acetylen
1-(4-Hexylphenyl)-2-(5-hexyl-pyrimidin-2-yl)-acetylen
1-(4-Hexylphenyl)-2-(5-heptyl-pyrimidin-2-yl)-acetylen
1-(4-Hexylphenyl)-2-(5-octyl-pyrimidin-2-yl)-acetylen
1-(4-Propyloxyphenyl)-2-(5-methyl-pyrimidin-2-yl)-acetylen
1-(4-Propyloxyphenyl)-2-(5-ethyl-pyrimidin-2-yl)-acetylen
1-(4-Propyloxyphenyl)-2-(5-propyl-pyrimidin-2-yl)-acetylen
1-(4-Propyloxyphenyl)-2-(5-butyl-pyrimidin-2-yl)-acetylen
1-(4-Propyloxyphenyl)-2-(5-pentyl-pyrimidin-2-yl)-acetylen
1-(4-Propyloxyphenyl)-2-(5-hexyl-pyrimidin-2-yl)-acetylen
1-(4-Propyloxyphenyl)-2-(5-heptyl-pyrimidin-2-yl)-acetylen
1-(4-Propyloxyphenyl)-2-(5-octyl-pyrimidin-2-yl)-acetylen
1-(4-Butyloxyphenyl)-2-(5-methyl-pyrimidin-2-yl)-acetylen
1-(4-Butyloxyphenyl)-2-(5-ethyl-pyrimidin-2-yl)-acetylen
1-(4-Butyloxyphenyl)-2-(5-propyl-pyrimidin-2-yl)-acetylen
1-(4-Butyloxyphenyl)-2-(5-butyl-pyrimidin-2-yl)-acetylen
1-(4-Butyloxyphenyl)-2-(5-pentyl-pyrimidin-2-yl)-acetylen
1-(4-Butyloxyphenyl)-2-(5-hexyl-pyrimidin-2-yl)-acetylen
1-(4-Butyloxyphenyl)-2-(5-heptyl-pyrimidin-2-yl)-acetylen
1-(4-Butyloxyphenyl)-2-(5-octyl-pyrimidin-2-yl)-acetylen
1-(4-Pentyloxyphenyl)-2-(5-methyl-pyrimidin-2-yl)-acetylen
1-(4-Pentyloxyphenyl)-2-(5-ethyl-pyrimidin-2-yl)-acetylen
1-(4-Pentyloxyphenyl)-2-(5-propyl-pyrimidin-2-yl)-acetylen
1-(4-Pentyloxyphenyl)-2-(5-butyl-pyrimidin-2-yl)-acetylen
1-(4-Pentyloxyphenyl)-2-(5-pentyl-pyrimidin-2-yl)-acetylen
1-(4-Pentyloxyphenyl)-2-(5-hexyl-pyrimidin-2-yl)-acetylen
1-(4-Pentyloxyphenyl)-2-(5-heptyl-pyrimidin-2-yl)-acetylen
1-(4-Pentyloxyphenyl)-2-(5-octyl-pyrimidin-2-yl)-acetylen
1-(4-Propylphenyl)-2-(5-methyloxy-pyrimidin-2-yl)-acetylen
1-(4-Propylphenyl)-2-(5-ethyloxy-pyrimidin-2-yl)-acetylen
1-(4-Propylphenyl)-2-(5-propyloxy-pyrimidin-2-yl)-acetylen
1-(4-Propylphenyl)-2-(5-butyloxy-pyrimidin-2-yl)-acetylen

1-(4-Propylphenyl)-2-(5-pentyloxy-pyrimidin-2-yl)-acetylen
1-(4-Propylphenyl)-2-(5-hexyloxy-pyrimidin-2-yl)-acetylen
1-(4-Propylphenyl)-2-(5-heptyloxy-pyrimidin-2-yl)-acetylen
1-(4-Propylphenyl)-2-(5-octyloxy-pyrimidin-2-yl)-acetylen
1-(4-Butylphenyl)-2-(5-methyloxy-pyrimidin-2-yl)-acetylen
1-(4-Butylphenyl)-2-(5-ethyloxy-pyrimidin-2-yl)-acetylen
1-(4-Butylphenyl)-2-(5-propyloxy-pyrimidin-2-yl)-acetylen
1-(4-Butylphenyl)-2-(5-butyloxy-pyrimidin-2-yl)-acetylen
1-(4-Butylphenyl)-2-(5-pentyloxy-pyrimidin-2-yl)-acetylen
1-(4-Butylphenyl)-2-(5-hexyloxy-pyrimidin-2-yl)-acetylen
1-(4-Butylphenyl)-2-(5-heptyloxy-pyrimidin-2-yl)-acetylen
1-(4-Butylphenyl)-2-(5-octyloxy-pyrimidin-2-yl)-acetylen
1-(4-Pentylphenyl)-2-(5-methyloxy-pyrimidin-2-yl)-acetylen
1-(4-Pentylphenyl)-2-(5-ethyloxy-pyrimidin-2-yl)-acetylen
1-(4-Pentylphenyl)-2-(5-propyloxy-pyrimidin-2-yl)-acetylen
1-(4-Pentylphenyl)-2-(5-butyloxy-pyrimidin-2-yl)-acetylen
1-(4-Pentylphenyl)-2-(5-pentyloxy-pyrimidin-2-yl)-acetylen
1-(4-Pentylphenyl)-2-(5-hexyloxy-pyrimidin-2-yl)-acetylen
1-(4-Pentylphenyl)-2-(5-heptyloxy-pyrimidin-2-yl)-acetylen
1-(4-Pentylphenyl)-2-(5-octyloxy-pyrimidin-2-yl)-acetylen
1-(4-Hexylphenyl)-2-(5-methyloxy-pyrimidin-2-yl)acetylen
1-(4-Hexylphenyl)-2-(5-ethyloxy-pyrimidin-2-yl)-acetylen
1-(4-Hexylphenyl)-2-(5-propyloxy-pyrimidin-2-yl)-acetylen
1-(4-Hexpylphenyl)-2-(5-butyloxy-pyrimidin-2-yl)-acetylen
1-(4-Hexylphenyl)-2-(5-pentyloxy-pyrimidin-2-yl)acetylen
1-(4-Hexylphenyl)-2-(5-hexyloxy-pyrimidin-2-yl)-acetylen
1-(4-Hexylphenyl)-2-(5-heptyloxy-pyrimidin-2-yl)-acetylen
1-(4-Hexylphenyl)-2-(5-octyloxy-pyrimidin-2-yl)-acetylen
1-[4-(trans-4-Propylcyclohexyl)phenyl]-2-(5-methylpyridin-2-yl)-acetylen; F. 137°, K. 217°
1-[4-(trans-4-Propylcyclohexyl)phenyl]-2-(5-ethylpyridin-2-yl)-acetylen
1-[4-(trans-4-Propylcyclohexyl)phenyl]-2-(5-propylpyridin-2-yl)-acetylen
1-[4-(trans-4-Propylcyclohexyl)phenyl]-2-(5-butylpyridin-2-yl)-acetylen
1-[4-(trans-4-Propylcyclohexyl)phenyl]-2-(5-pentylpyridin-2-yl)-acetylen
1-[4-(trans-4-Propylcyclohexyl)phenyl]-2-(5-hexylpyridin-2-yl)-acetylen
1-[4-(trans-4-Butylcyclohexyl)phenyl]-2-(5-methylpyridin-2-yl)-acetylen
1-[4-(trans-4-Butylcyclohexyl)phenyl]-2-(5-ethylpyridin-2-yl)-acetylen
1-[4-(trans-4-Butylcyclohexyl)phenyl]-2-(5-propylpyridin-2-yl)-acetylen
1-[4-(trans-4-Butylcyclohexyl)phenyl]-2-(5-butylpyridin-2-yl)-acetylen
1-[4-(trans-4-Butylcyclohexyl)phenyl]-2-(5-pentylpyridin-2-yl)-acetylen
1-[4-(trans-4-Butylcyclohexyl)phenyl]-2-(5-hexylpyridin-2-yl)-acetylen
1-[4-(trans-4-Pentylcyclohexyl)phenyl]-2-(5-methylpyridin-2-yl)-acetylen
1-[4-(trans-4-Pentylcyclohexyl)phenyl]-2-(5-ethylpyridin-2-yl)-acetylen
1-[4-(trans-4-Pentylcyclohexyl)phenyl]-2-(5-propylpyridin-2-yl)-acetylen
1-[4-(trans-4-Pentylcyclohexyl)phenyl]-2-(5-butylpyridin-2-yl)-acetylen
1-[4-(trans-4-Pentylcyclohexyl)phenyl]-2-(5-pentylpyridin-2-yl)-acetylen
1-[4-(trans-4-Pentylcyclohexyl)phenyl]-2-(5-hexylpyridin-2-yl)-acetylen
1-[4-(trans-4-Pentylcyclohexyl)phenyl]-2-(5-heptylpyridin-2-yl)-acetylen
1-[4-(trans-4-Hexylcyclohexyl)phenyl]-2-(5-methylpyridin-2-yl)-acetylen
1-[4-(trans-4-Hexylcyclohexyl)phenyl]-2-(5-ethylpyridin-2-yl)-acetylen
1-[4-(trans-4-Hexylcyclohexyl)phenyl]-2-(5-propylpyridin-2-yl)-acetylen
1-[4-(trans-4-Hexylcyclohexyl)phenyl]-2-(5-butylpyridin-2-yl)-acetylen
1-[4-(trans-4-Hexylcyclohexyl)phenyl]-2-(5-pentylpyridin-2-yl)-acetylen
1-[4-(trans-4-Hexylcyclohexyl)phenyl]-2-(5-hexylpyridin-2-yl)-acetylen
1-[4-(trans-4-Propylcyclohexyl)phenyl]-2-(5-methyloxypyridin-2-yl)-acetylen
1-[4-(trans-4-Propylcyclohexyl)phenyl]-2-(5-ethyloxypyridin-2-yl)-acetylen
1-[4-(trans-4-Propylcyclohexyl)phenyl]-2-(5-propyloxypyridin-2-yl)-acetylen
1-[4-(trans-4-Propylcyclohexyl)phenyl]-2-(5-butyloxypyridin-2-yl)-acetylen
1-[4-(trans-4-Propylcyclohexyl)phenyl]-2-(5-pentyloxypyridin-2-yl)-acetylen

1-[4-(trans-4-Propylcyclohexyl)phenyl]-2-(5-hexyloxypyridin-2-yl)-acetylen
1-[4-(trans-4-Butylcyclohexyl)phenyl]-2-(5-methyloxypyridin-2-yl)-acetylen
1-[4-(trans-4-Butylcyclohexyl)phenyl]-2-(5-ethyloxypyridin-2-yl)-acetylen
1-[4-(trans-4-Butylcyclohexyl)phenyl]-2-(5-propyloxypyridin-2-yl)-acetylen
1-[4-(trans-4-Butylcyclohexyl)phenyl]-2-(5-butyloxypyridin-2-yl)-acetylen
1-[4-(trans-4-Butylcyclohexyl)phenyl]-2-(5-pentyloxypyridin-2-yl)-acetylen
1-[4-(trans-4-Pentylcyclohexyl)phenyl]-2-(5-methyloxypyridin-2-yl)-acetylen
1-[4-(trans-4-Pentylcyclohexyl)phenyl]-2-(5-ethyloxypyridin-2-yl)-acetylen
1-[4-(trans-4-Pentylcyclohexyl)phenyl]-2-(5-propyloxypyridin-2-yl)-acetylen
1-[4-(trans-4-Pentylcyclohexyl)phenyl]-2-(5-butyloxypyridin-2-yl)-acetylen
1-[4-(trans-4-Pentylcyclohexyl)phenyl]-2-(5-pentyloxypyridin-2-yl)-acetylen
1-[4-(trans-4-Pentylcyclohexyl)phenyl]-2-(5-hexyloxypyridin-2-yl)-acetylen
1-[4-(trans-4-Ethylcyclohexyl)phenyl]-2-(5-methyloxypyridin-2-yl)-acetylen
1-[4-(trans-4-Ethylcyclohexyl)phenyl]-2-(5-ethyloxypyridin-2-yl)-acetylen
1-[4-(trans-4-Ethylcyclohexyl)phenyl]-2-(5-propyloxypyridin-2-yl)-acetylen
1-[4-(trans-4-Ethylcyclohexyl)phenyl]-2-(5-butyloxypyridin-2-yl)-acetylen
1-[4-(trans-4-Ethylcyclohexyl)phenyl]-2-(5-pentyloxypyridin-2-yl)-acetylen
1-[4-(trans-4-Ethylcyclohexyl)phenyl]-2-(5-hexyloxypyridin-2-yl)-acetylen
1-[4-(trans-4-Propylcyclohexyl)phenyl]-2-(5-methylpyrimidin-2-yl)-acetylen
1-[4-(trans-4-Propylcyclohexyl)phenyl]-2-(5-ethylpyrimidin-2-yl)-acetylen
1-[4-(trans-4-Propylcyclohexyl)phenyl]-2-(5-propylpyrimidin-2-yl)-acetylen
1-[4-(trans-4-Propylcyclohexyl)phenyl]-2-(5-butylpyrimidin-2-yl)-acetylen
1-[4-(trans-4-Propylcyclohexyl)phenyl]-2-(5-pentylpyrimidin-2-yl)-acetylen
1-[4-(trans-4-Propylcyclohexyl)phenyl]-2-(5-hexylpyrimidin-2-yl)-acetylen
1-[4-(trans-4-Propylcyclohexyl)phenyl]-2-(5-heptylpyrimidin-2-yl)-acetylen
1-[4-(trans-4-Propylcyclohexyl)phenyl]-2-(5-octylpyrimidin-2-yl)-acetylen
1-[4-(trans-4-Butylcyclohexyl)phenyl]-2-(5-methylpyrimidin-2-yl)-acetylen
1-[4-(trans-4-Butylcyclohexyl)phenyl]-2-(5-ethylpyrimidin-2-yl)-acetylen
1-[4-(trans-4-Butylcyclohexyl)phenyl]-2-(5-propylpyrimidin-2-yl)-acetylen
1-[4-(trans-4-Butylcyclohexyl)phenyl]-2-(5-butylpyrimidin-2-yl)-acetylen
1-[4-(trans-4-Butylcyclohexyl)phenyl]-2-(5-pentylpyrimidin-2-yl)-acetylen
1-[4-(trans-4-Butylcyclohexyl)phenyl]-2-(5-hexylpyrimidin-2-yl)-acetylen
1-[4-(trans-4-Pentylcyclohexyl)phenyl]-2-(5-methylpyrimidin-2-yl)-acetylen
1-[4-(trans-4-Pentylcyclohexyl)phenyl]-2-(5-ethylpyrimidin-2-yl)-acetylen
1-[4-(trans-4-Pentylcyclohexyl)phenyl]-2-(5-propylpyrimidin-2-yl)-acetylen
1-[4-(trans-4-Pentylcyclohexyl)phenyl]-2-(5-butylpyrimidin-2-yl)-acetylen
1-[4-(trans-4-Pentylcyclohexyl)phenyl]-2-(5-pentylpyrimidin-2-yl)-acetylen
1-[4-(trans-4-Pentylcyclohexyl)phenyl]-2-(5-hexylpyrimidin-2-yl)-acetylen
1-[4-(trans-4-Ethylcyclohexyl)phenyl]-2-(5-methylpyrimidin-2-yl)-acetylen
1-[4-(trans-4-Ethylcyclohexyl)phenyl]-2-(5-ethylpyrimidin-2-yl)-acetylen
1-[4-(trans-4-Ethylcyclohexyl)phenyl]-2-(5-propylpyrimidin-2-yl)-acetylen
1-[4-(trans-4-Ethylcyclohexyl)phenyl]-2-(5-butylpyrimidin-2-yl)-acetylen
1-[4-(trans-4-Ethylcyclohexyl)phenyl]-2-(5-pentylpyrimidin-2-yl)-acetylen
1-[4-(trans-4-Ethylcyclohexyl)phenyl]-2-(5-hexylpyrimidin-2-yl)-acetylen
1-[4-(trans-4-Propylcyclohexyl)phenyl]-2-(5-methyloxypyrimidin-2-yl)-acetylen
1-[4-(trans-4-Propylcyclohexyl)phenyl]-2-(5-ethyloxypyrimidin-2-yl)-acetylen
1-[4-(trans-4-Propylcyclohexyl)phenyl]-2-(5-propyloxypyrimidin-2-yl)-acetylen
1-[4-(trans-4-Propylcyclohexyl)phenyl]-2-(5-butyloxypyrimidin-2-yl)-acetylen
1-[4-(trans-4-Propylcyclohexyl)phenyl]-2-(5-pentyloxypyrimidin-2-yl)-acetylen
1-[4-(trans-4-Propylcyclohexyl)phenyl]-2-(5-hexyloxypyrimidin-2-yl)-acetylen
1-[4-(trans-4-Propylcyclohexyl)phenyl]-2-(5-heptyloxypyrimidin-2-yl)-acetylen
1-[4-(trans-4-Propylcyclohexyl)phenyl]-2-(5-octyloxypyrimidin-2-yl)-acetylen
1-[4-(trans-4-Butylcyclohexyl)phenyl]-2-(5-methyloxypyrimidin-2-yl)-acetylen
1-[4-(trans-4-Butylcyclohexyl)phenyl]-2-(5-ethyloxypyrimidin-2-yl)-acetylen
1-[4-(trans-4-Butylcyclohexyl)phenyl]-2-(5-propyloxypyrimidin-2-yl)-acetylen
1-[4-(trans-4-Butylcyclohexyl)phenyl]-2-(5-butyloxypyrimidin-2-yl)-acetylen
1-[4-(trans-4-Butylcyclohexyl)phenyl]-2-(5-pentyloxypyrimidin-2-yl)-acetylen
1-[4-(trans-4-Ethylcyclohexyl)phenyl]-2-(5-methyloxypyrimidin-2-yl)-acetylen

1-[4-(trans-4-Ethylcyclohexyl)phenyl]-2-(5-ethyloxypyrimidin-2-yl)-acetylen
1-[4-(trans-4-Ethylcyclohexyl)phenyl]-2-(5-propyloxypyrimidin-2-yl)-acetylen
1-[4-(trans-4-Ethylcyclohexyl)phenyl]-2-(5-butyloxypyrimidin-2-yl)-acetylen
1-[4-(trans-4-Ethylcyclohexyl)phenyl]-2-(5-pentyloxypyrimidin-2-yl)-acetylen
1-[4-(trans-4-Ethylcyclohexyl)phenyl]-2-(5-hexyloxypyrimidin-2-yl)-acetylen
1-[4-(trans-4-Methylcyclohexyl)phenyl]-2-(5-methyloxypyrimidin-2-yl)-acetylen
1-[4-(trans-4-Methylcyclohexyl)phenyl]-2-(5-ethyloxypyrimidin-2-yl)-acetylen
1-[4-(trans-4-Methylcyclohexyl)phenyl]-2-(5-propyloxypyrimidin-2-yl)-acetylen
1-[4-(trans-4-Methylcyclohexyl)phenyl]-2-(5-butyloxypyrimidin-2-yl)-acetylen
1-[4-(trans-4-Methylcyclohexyl)phenyl]-2-(5-pentyloxypyrimidin-2-yl)-acetylen
1-[4-(trans-4-Methylcyclohexyl)phenyl]-2-(5-hexyloxypyrimidin-2-yl)-acetylen

Beispiel 3

Analog Beispiel 1 erhält man durch Umsetzung von p-Propylphenylacetylen (herstellbar z.B. analog Smith, Hoehn, Amer. Soc. 63 1175 (1941)) und 2-Brom-5-chlor-pyridin in Gegenwart von Bis-(triphenylphosphin)-palladium(II)-chlorid und Kupfer(I)-jodid 1-(p-Propylphenyl)-2-(5-chlor-pyridin-2-yl)-acetylen.

Analog werden hergestellt:
1-(p-Methylphenyl)-2-(5-chlor-pyridin-2-yl)-acetylen
1-(p-Ethylphenyl)-2-(5-chlor-pyridin-2-yl)-acetylen
1-(p-Butylphenyl)-2-(5-chlor-pyridin-2-yl)-acetylen
1-(p-Pentylphenyl)-2-(5-chlor-pyridin-2-yl)-acetylen
1-(p-Hexylphenyl)-2-(5-chlor-pyridin-2-yl)-acetylen
1-(p-Methoxyphenyl)-2-(5-chlor-pyridin-2-yl)-acetylen
1-(p-Ethoxyphenyl)-2-(5-chlor-pyridin-2-yl)-acetylen
1-(p-Propoxyphenyl)-2-(5-chlor-pyridin-2-yl)-acetylen
1-(p-Butyloxyphenyl)-2-(5-chlor-pyridin-2-yl)-acetylen
1-(p-Pentyloxyphenyl)-2-(5-chlor-pyridin-2-yl)-acetylen
1-(p-Hexyloxyphenyl)-2-(5-chlor-pyridin-2-yl)-acetylen
1-(p-Methylphenyl)-2-(5-chlor-pyrimidin-2-yl)-acetylen
1-(p-Ethylphenyl)-2-(5-chlor-pyrimidin-2-yl)-acetylen
1-(p-Propylphenyl)-2-(5-chlor-pyrimidin-2-yl)-acetylen
1-(p-Butylphenyl)-2-(5-chlor-pyrimidin-2-yl)-acetylen
1-(p-Pentylphenyl)-2-(5-chlor-pyrimidin-2-yl)-acetylen
1-(p-Hexylphenyl)-2-(5-chlor-pyrimidin-2-yl)-acetylen
1-(p-Methoxyphenyl)-2-(5-chlor-pyrimidin-2-yl)-acetylen
1-(p-Ethoxyphenyl)-2-(5-chlor-pyrimidin-2-yl)-acetylen
1-(p-Propoxyphenyl)-2-(5-chlor-pyrimidin-2-yl)-acetylen
1-(p-Butyloxyphenyl)-2-(5-chlor-pyrimidin-2-yl)-acetylen
1-(p-Pentyloxyphenyl)-2-(5-chlor-pyrimidin-2-yl)-acetylen
1-(p-Hexyloxyphenyl)-2-(5-chlor-pyrimidin-2-yl)-acetylen

Beispiel 4

Analog Beispiel 1 erhält man durch Umsetzung von 0,02 mol trans-4-Pentylcyclohexylacetylen und 0,01 mol 1,4-Dibrombenzol das entsprechende 1-(trans-4-Pentylcyclohexyl)-2-[4-{2-(trans-4-pentylcyclohexyl)-ethinyl}-phenyl]-acetylen.

Analog werden hergestellt:
1-(trans-4-Ethylcyclohexyl)-2-[4-{2-(trans-4-ethylcyclohexyl)-ethinyl}-phenyl]-acetylen
1-(trans-4-Propylcyclohexyl)-2-[4-{2-(trans-4-propylcyclohexyl)-ethinyl}-phenyl]-acetylen
1-(trans-4-Butylcyclohexyl)-2-[4-{2-(trans-4-butylcyclohexyl)-ethinyl}-phenyl]-acetylen
1-(trans-4-Hexylcyclohexyl)-2-[4-{2-(trans-4-hexylcyclohexyl)-ethinyl}-phenyl]-acetylen
1-(4-Ethoxyphenyl)-2-[5-{2-(4-ethoxyphenyl)-ethinyl}-pyridin-2-yl]-acetylen
1-(4-Propoxyphenyl)-2-[5-{2-(4-propoxyphenyl)-ethinyl}-pyridin-2-yl]-acetylen
1-(4-Butyloxyphenyl)-2-[5-{2-(4-butyloxyphenyl)-ethinyl}-pyridin-2-yl]-acetylen
1-(4-Pentyloxyphenyl)-2-[5-{2-(4-pentyloxyphenyl)-ethinyl}-pyridin-2-yl]-acetylen
1-(4-Methoxyphenyl)-2-[5-{2-(4-methoxyphenyl)-ethinyl}-pyridin-2-yl]-acetylen

1-(4-Ethoxyphenyl)-2-[5-{2-(4-ethoxyphenyl)-ethiny}-pyrimidin-2-yl]-acetylen
1-(4-Propoxyphenyl)-2-[5-{2-(4-propoxyphenyl)-ethinyl}-pyrimidin-2-yl]-acetylen
1-(4-Butyloxyphenyl)-2-[5-{2-(4-butyloxyphenyl)-ethinyl}-pyrimidin-2-yl]-acetylen
1-(4-Pentyloxyphenyl)-2-[5-{2-(4-pentyloxyphenyl)-ethinyl}-pyrimidin-2-yl]-acetylen
1-(4-Hexyloxyphenyl)-2-[5-{2-(4-hexyloxyphenyl)-ethinyl}-pyrimidin-2-yl]-acetylen
1-(4-Heptyloxyphenyl)-2-[5-{2-(4-heptyloxyphenyl)-ethinyl}-pyrimidin-2-yl]-acetylen
1-(4-Octyloxyphenyl)-2-[5-{2-(4-octyloxyphenyl)-ethinyl}-pyrimidin-2-yl]-acetylen.

Beispiel 5

Man versetzt ein Gemisch aus 0,1 mol Difluorphenetol, 0,1 mol Tetramethylethylendiamin (TMEDA) und 200 ml THF bei -70° bis -60° mit 0,105 mol n-Butyllithium (1,5 mol in n-Hexan). Man rührt noch 3 Stunden bei dieser Temperatur, gibt dann 0,1 mol $J_2$ in 150 ml THF zu. Anschliessend hydrolysiert man bei -20° mit einer 5%igen Natriumthiolsulfatlösung. Nach extraktiver Aufarbeitung wird das Rohprodukt durch Destillation im Vakuum gereinigt.

0,01 mol dieser Jodverbindung werden mit 0,01 mol trans-4-Pentylcyclohexylacetylen, 30 ml Triethylamin, 0,2 mmol Pd-Katalysator und 0,1 mmol CuJ 15 Stunden auf 60° bis 70° erwärmt. Das Reaktionsgemisch wird mit Petrolether verdünnt und filtriert. Man erhält nach Eindampfen und Reinigung 1-(trans-4-Pentylcyclohexyl)-2-(2,3-difluor-4-ethoxyphenyl)-acetylen.

Analog werden hergestellt:
1-(trans-4-Methylcyclohexyl)-2-(2,3-difluor-4-ethoxyphenyl)-acetylen
1-(trans-4-Ethylcyclohexyl)-2-(2,3-difluor-4-ethoxyphenyl)-acetylen
1-(trans-4-Propylcyclohexyl)-2-(2,3-difluor-4-ethoxyphenyl)-acetylen
1-(trans-4-Butylcyclohexyl)-2-(2,3-difluor-4-ethoxyphenyl)-acetylen
1-(trans-4-Hexylcyclohexyl)-2-(2,3-difluor-4-ethoxyphenyl)-acetylen
1-(trans-4-Heptylcyclohexyl)-2-(2,3-difluor-4-ethoxyphenyl)-acetylen
1-(trans-4-Methylcyclohexyl)-2-(2,3-difluor-4-methoxyphenyl)-acetylen
1-(trans-4-Ethylcyclohexyl)-2-(2,3-difluor-4-methoxyphenyl)-acetylen
1-(trans-4-Propylcyclohexyl)-2-(2,3-difluor-4-methoxyphenyl)-acetylen
1-(trans-4-Butylcyclohexyl)-2-(2,3-difluor-4-methoxyphenyl)-acetylen
1-(trans-4-Pentylcyclohexyl)-2-(2,3-difluor-4-methoxyphenyl)-acetylen
1-(trans-4-Hexylcyclohexyl)-2-(2,3-difluor-4-methoxyphenyl)-acetylen
1-(trans-4-Heptylcyclohexyl)-2-(2,3-difluor-4-methoxyphenyl)-acetylen
1-(trans-4-Methylcyclohexyl)-2-(2,3-difluor-4-propoxyphenyl)-acetylen
1-(trans-4-Ethylcyclohexyl)-2-(2,3-difluor-4-propoxyphenyl)-acetylen
1-(trans-4-Propylcyclohexyl)-2-(2,3-difluor-4-propoxyphenyl)-acetylen
1-(trans-4-Butylcyclohexyl)-2-(2,3-difluor-4-propoxyphenyl)-acetylen
1-(trans-4-Pentylcyclohexyl)-2-(2,3-difluor-4-propoxyphenyl)-acetylen
1-(trans-4-Hexylcyclohexyl)-2-(2,3-difluor-4-propoxyphenyl)-acetylen
1-(trans-4-Heptylcyclohexyl)-2-(2,3-difluor-4-propoxyphenyl)-acetylen
1-(trans-4-Methylcyclohexyl)-2-(2,3-difluor-4-butoxyphenyl)-acetylen
1-(trans-4-Ethylcyclohexyl)-2-(2,3-difluor-4-butoxyphenyl)-acetylen
1-(trans-4-Propylcyclohexyl)-2-(2,3-difluor-4-butoxyphenyl)-acetylen
1-(trans-4-Butylcyclohexyl)-2-(2,3-difluor-4-butoxyphenyl)-acetylen
1-(trans-4-Pentylcyclohexyl)-2-(2,3-difluor-4-butoxyphenyl)-acetylen
1-(trans-4-Hexylcyclohexyl)-2-(2,3-difluor-4-butoxyphenyl)-acetylen
1-(trans-4-Heptylcyclohexyl)-2-(2,3-difluor-4-butoxyphenyl)-acetylen
1-(trans-4-Methylcyclohexyl)-2-(2,3-difluor-4-pentyloxyphenyl)-acetylen
1-(trans-4-Ethylcyclohexyl)-2-(2,3-difluor-4-pentyloxyphenyl)-acetylen
1-(trans-4-Propylcyclohexyl)-2-(2,3-difluor-4-pentyloxyphenyl)-acetylen
1-(trans-4-Butylcyclohexyl)-2-(2,3-difluor-4-pentyloxyphenyl)-acetylen
1-(trans-4-Pentylcyclohexyl)-2-(2,3-difluor-4-pentyloxyphenyl)-acetylen
1-(trans-4-Hexylcyclohexyl)-2-(2,3-difluor-4-pentyloxyphenyl)-acetylen
1-(trans-4-Heptylcyclohexyl)-2-(2,3-difluor-4-pentyloxyphenyl)-acetylen
1-(trans-4-Methylcyclohexyl)-2-(2,3-difluor-4-hexyloxyphenyl)-acetylen
1-(trans-4-Ethylcyclohexyl)-2-(2,3-difluor-4-hexyloxyphenyl)-acetylen
1-(trans-4-Propylcyclohexyl)-2-(2,3-difluor-4-hexyloxyphenyl)-acetylen
1-(trans-4-Butylcyclohexyl)-2-(2,3-difluor-4-hexyloxyphenyl)-acetylen

1-(trans-4-Pentylcyclohexyl)-2-(2,3-difluor-4-hexyloxyphenyl)-acetylen
1-(trans-4-Hexylcyclohexyl)-2-(2,3-difluor-4-hexyloxyphenyl)-acetylen
1-(trans-4-Heptylcyclohexyl)-2-(2,3-difluor-4-hexyloxyphenyl)-acetylen
1-[trans-4-(4-Methylphenyl)cyclohexyl]-2-(2,3-difluor-4-methoxyphenyl)-acetylen
1-[trans-4-(4-Ethylphenyl)cyclohexyl]-2-(2,3-difluor-4-methoxyphenyl)-acetylen
1-[trans-4-(4-Propylphenyl)cyclohexyl]-2-(2,3-difluor-4-methoxyphenyl)-acetylen
1-[trans-4-(4-Butylphenyl)cyclohexyl]-2-(2,3-difluor-4-methoxyphenyl)-acetylen
1-[trans-4-(4-Pentylphenyl)cyclohexyl]-2-(2,3-difluor-4-methoxyphenyl)-acetylen
1-[trans-4-(4-Hexylphenyl)cyclohexyl]-2-(2,3-difluor-4-methoxyphenyl)-acetylen
1-[trans-4-(4-Heptylphenyl)cyclohexyl]-2-(2,3-difluor-4-methoxyphenyl)-acetylen
1-[trans-4-(4-Methylphenyl)cyclohexyl]-2-(2,3-difluor-4-propoxyphenyl)-acetylen
1-[trans-4-(4-Ethylphenyl)cyclohexyl]-2-(2,3-difluor-4-propoxyphenyl)-acetylen
1-[trans-4-(4-Propylphenyl)cyclohexyl]-2-(2,3-difluor-4-propoxyphenyl)-acetylen
1-[trans-4-(4-Butylphenyl)cyclohexyl]-2-(2,3-difluor-4-propoxyphenyl)-acetylen
1-[trans-4-(4-Pentylphenyl)cyclohexyl]-2-(2,3-difluor-4-propoxyphenyl)-acetylen
1-[trans-4-(4-Hexylphenyl)cyclohexyl]-2-(2,3-difluor-4-propoxyphenyl)-acetylen
1-[trans-4-(4-Heptylphenyl)cyclohexyl]-2-(2,3-difluor-4-propoxyphenyl)-acetylen
1-[trans-4-(4-Methylphenyl)cyclohexyl]-2-(2,3-difluor-4-butoxyphenyl)-acetylen
1-[trans-4-(4-Ethylphenyl)cyclohexyl]-2-(2,3-difluor-4-butoxyphenyl)-acetylen
1-[trans-4-(4-Propylphenyl)cyclohexyl]-2-(2,3-difluor-4-butoxyphenyl)-acetylen
1-[trans-4-(4-Butylphenyl)cyclohexyl]-2-(2,3-difluor-4-butoxyphenyl)-acetylen
1-[trans-4-(4-Pentylphenyl)cyclohexyl]-2-(2,3-difluor-4-butoxyphenyl)-acetylen
1-[trans-4-(4-Hexylphenyl)cyclohexyl]-2-(2,3-difluor-4-butoxyphenyl)-acetylen
1-[trans-4-(4-Heptylphenyl)cyclohexyl]-2-(2,3-difluor-4-butoxyphenyl)-acetylen
1-[trans-4-(4-Methylphenyl)cyclohexyl]-2-(2,3-difluor-4-pentyloxyphenyl)-acetylen
1-[trans-4-(4-Ethylphenyl)cyclohexyl]-2-(2,3-difluor-4-pentyloxyphenyl)-acetylen
1-[trans-4-(4-Propylphenyl)cyclohexyl]-2-(2,3-difluor-4-pentyloxyphenyl)-acetylen
1-[trans-4-(4-Butylphenyl)cyclohexyl]-2-(2,3-difluor-4-pentyloxyphenyl)-acetylen
1-[trans-4-(4-Pentylphenyl)cyclohexyl]-2-(2,3-difluor-4-pentyloxyphenyl)-acetylen
1-[trans-4-(4-Hexylphenyl)cyclohexyl]-2-(2,3-difluor-4-pentyloxyphenyl)-acetylen
1-[trans-4-(4-Heptylphenyl)cyclohexyl]-2-(2,3-difluor-4-pentyloxyphenyl)-acetylen
1-[trans-4-(trans-4-Ethylcyclohexyl)cyclohexyl]-2-(2,3-difluor-4-ethoxyphenyl)-acetylen
1-[trans-4-(trans-4-Propylcyclohexyl)cyclohexyl]-2-(2,3-difluor-4-ethoxyphenyl)-acetylen
1-[trans-4-(trans-4-Butylcyclohexyl)cyclohexyl]-2-(2,3-difluor-4-ethoxyphenyl)-acetylen
1-[trans-4-(trans-4-Pentylcyclohexyl)cyclohexyl]-2-(2,3-difluor-4-ethoxyphenyl)-acetylen
1-[trans-4-(trans-4-Ethylcyclohexyl)cyclohexyl]-2-(2,3-difluor-4-propoxyphenyl)-acetylen
1-[trans-4-(trans-4-Propylcyclohexyl)cyclohexyl]-2-(2,3-difluor-4-propoxyphenyl)-acetylen
1-[trans-4-(trans-4-Butylcyclohexyl)cyclohexyl]-2-(2,3-difluor-4-propoxyphenyl)-acetylen
1-[trans-4-(trans-4-Pentylcyclohexyl)cyclohexyl]-2-(2,3-difluor-4-propoxyphenyl)-acetylen
1-[trans-4-(trans-4-Ethylcyclohexyl)cyclohexyl]-2-(2,3-difluor-4-butoxyphenyl)-acetylen
1-[trans-4-(trans-4-Propylcyclohexyl)cyclohexyl]-2-(2,3-difluor-4-butoxyphenyl)-acetylen
1-[trans-4-(trans-4-Butylcyclohexyl)cyclohexyl]-2-(2,3-difluor-4-butoxyphenyl)-acetylen
1-[trans-4-(trans-4-Pentylcyclohexyl)cyclohexyl]-2-(2,3-difluor-4-butoxyphenyl)-acetylen
1-[trans-4-(trans-4-Ethylcyclohexyl)cyclohexyl]-2-(2,3-difluor-4-pentyloxyphenyl)-acetylen
1-[trans-4-(trans-4-Propylcyclohexyl)cyclohexyl]-2-(2,3-difluor-4-pentyloxyphenyl)-acetylen
1-[trans-4-(trans-4-Butylcyclohexyl)cyclohexyl]-2-(2,3-difluor-4-pentyloxyphenyl)-acetylen
1-[trans-4-(trans-4-Pentylcyclohexyl)cyclohexyl]-2-(2,3-difluor-4-pentyloxyphenyl)-acetylen

Beispiel 6

Ein Gemisch aus 0,06 mol trans-4-Pentylcyclohexyl-acetylen, 0,06 mol 4-Trifluormethyl-brombenzol, 250 ml Triethylamin, 1,2 mmol Pd(II)-Katalysator und 0,6 mmol CuJ wird 15 Stunden bei Raumtemperatur gerührt. Man verdünnt das Reaktionsgemisch mit Petrolether und filtriert. Nach Eindampfen und Reinigung erhält man 1-(trans-4-Pentylcyclohexyl)-2-(4-trifluormethylphenyl)-acetylen mit F. = 43°.

Analog werden hergestellt:

1-(trans-4-Methylcyclohexyl)-2-(4-trifluormethylphenyl)-acetylen
1-(trans-4-Ethylcyclohexyl)-2-(4-trifluormethylphenyl)-acetylen
1-(trans-4-Propylcyclohexyl)-2-(4-trifluormethylphenyl)-acetylen

1-(trans-4-Butylcyclohexyl)-2-(4-trifluormethylphenyl)-acetylen
1-(trans-4-Hexylcyclohexyl)-2-(4-trifluormethylphenyl)-acetylen
1-(trans-4-Heptylcyclohexyl)-2-(4-trifluormethylphenyl)-acetylen
1-(trans-4-Methylcyclohexyl)-2-(4-pentafluorethylphenyl)-acetylen
1-(trans-4-Ethylcyclohexyl)-2-(4-pentafluorethylphenyl)-acetylen
1-(trans-4-Propylcyclohexyl)-2-(4-pentafluorethylphenyl)-acetylen
1-(trans-4-Butylcyclohexyl)-2-(4-pentafluorethylphenyl)-acetylen
1-(trans-4-Pentylcyclohexyl)-2-(4-pentafluorethylphenyl)-acetylen
1-(trans-4-Hexylcyclohexyl)-2-(4-pentafluorethylphenyl)-acetylen
1-(trans-4-Heptylcyclohexyl)-2-(4-pentafluorethylphenyl)-acetylen
1-[trans-4-(4-Methylphenyl)cyclohexyl]-2-(4-trifluormethylphenyl)-acetylen
1-[trans-4-(4-Ethylphenyl)cyclohexyl]-2-(4-trifluormethylphenyl)-acetylen
1-[trans-4-(4-Propylphenyl)cyclohexyl]-2-(4-trifluormethylphenyl)-acetylen
1-[trans-4-(4-Butylphenyl)cyclohexyl]-2-(4-trifluormethylphenyl)-acetylen
1-[trans-4-(4-Pentylphenyl)cyclohexyl]-2-(4-trifluormethylphenyl)-acetylen
1-[trans-4-(4-Hexylphenyl)cyclohexyl]-2-(4-trifluormethylphenyl)-acetylen
1-[trans-4-(4-Heptylphenyl)cyclohexyl]-2-(4-trifluormethylphenyl)-acetylen
1-[trans-4-(trans-4-Methylcyclohexyl)cyclohexyl]-2-(4-trifluormethylphenyl)-acetylen
1-[trans-4-(trans-4-Ethylcyclohexyl)cyclohexyl]-2-(4-trifluormethylphenyl)-acetylen
1-[trans-4-(trans-4-Propylcyclohexyl)cyclohexyl]-2-(4-trifluormethylphenyl)-acetylen
1-[trans-4-(trans-4-Butylcyclohexyl)cyclohexyl]-2-(4-trifluormethylphenyl)-acetylen
1-[trans-4-(trans-4-Pentylcyclohexyl)cyclohexyl]-2-(4-trifluormethylphenyl)-acetylen
1-[trans-4-(trans-4-Hexylcyclohexyl)cyclohexyl]-2-(4-trifluormethylphenyl)-acetylen
1-[trans-4-(trans-4-Heptylcyclohexyl)cyclohexyl]-2-(4-trifluormethylphenyl)-acetylen

## Beispiel 7

Ein Gemisch aus 0,01 mol 5-Methylpyridin-2-yl-acetylen (herstellbar aus 5-Methyl-2-brompyridin durch Umsetzung mit Trimethylsilyl-acetylen und Pd-Katalysator und anschließende Abspaltung der Trimethylsilylgruppe), 0,01 mol 4-Jod-2,3-difluorphenetol (Darstellung siehe Beispiel 9), 30 ml Triethylamin, 0,2 mmol Pd-Katalysator und 0,1 mmol CuJ wird bei Raumtemperatur 24 Stunden lang gerührt. Das Reaktionsgemisch wird mit Petrolether verdünnt und über Kieselgel filtriert. Nach Eindampfen und Reinigung erhält man 1-(5-Methylpyridin-2-yl)-2-(2,3-difluor-4-ethoxyphenyl)-acetylen.

Analog werden hergestellt:

1-(5-Ethylpyridin-2-yl)-2-(2,3-difluor-4-ethoxyphenyl)-acetylen
1-(5-Propylpyridin-2-yl)-2-(2,3-difluor-4-ethoxyphenyl)-acetylen
1-(5-Butylpyridin-2-yl)-2-(2,3-difluor-4-ethoxyphenyl)-acetylen
1-(5-Pentylpyridin-2-yl)-2-(2,3-difluor-4-ethoxyphenyl)-acetylen
1-(5-Hexylpyridin-2-yl)-2-(2,3-difluor-4-ethoxyphenyl)-acetylen
1-(5-Heptylpyridin-2-yl)-2-(2,3-difluor-4-ethoxyphenyl)-acetylen
1-(5-Methylpyridin-2-yl)-2-(2,3-difluor-4-methoxyphenyl)-acetylen
1-(5-Ethylpyridin-2-yl)-2-(2,3-difluor-4-methoxyphenyl)-acetylen
1-(5-Propylpyridin-2-yl)-2-(2,3-difluor-4-methoxyphenyl)-acetylen
1-(5-Butylpyridin-2-yl)-2-(2,3-difluor-4-methoxyphenyl)-acetylen
1-(5-Pentylpyridin-2-yl)-2-(2,3-difluor-4-methoxyphenyl)-acetylen
1-(5-Hexylpyridin-2-yl)-2-(2,3-difluor-4-methoxyphenyl)-acetylen
1-(5-Heptylpyridin-2-yl)-2-(2,3-difluor-4-methoxyphenyl)-acetylen
1-(5-Methylpyridin-2-yl)-2-(2,3-difluor-4-propoxyphenyl)-acetylen
1-(5-Ethylpyridin-2-yl)-2-(2,3-difluor-4-propoxyphenyl)-acetylen
1-(5-Propylpyridin-2-yl)-2-(2,3-difluor-4-propoxyphenyl)-acetylen
1-(5-Butylpyridin-2-yl)-2-(2,3-difluor-4-propoxyphenyl)-acetylen
1-(5-Pentylpyridin-2-yl)-2-(2,3-difluor-4-propoxyphenyl)-acetylen
1-(5-Hexylpyridin-2-yl)-2-(2,3-difluor-4-propoxyphenyl)-acetylen
1-(5-Heptylpyridin-2-yl)-2-(2,3-difluor-4-propoxyphenyl)-acetylen
1-(5-Methylpyridin-2-yl)-2-(2,3-difluor-4-butoxyphenyl)-acetylen
1-(5-Ethylpyridin-2-yl)-2-(2,3-difluor-4-butoxyphenyl)-acetylen
1-(5-Propylpyridin-2-yl)-2-(2,3-difluor-4-butoxyphenyl)-acetylen
1-(5-Butylpyridin-2-yl)-2-(2,3-difluor-4-butoxyphenyl)-acetylen

1-(5-Pentylpyridin-2-yl)-2-(2,3-difluor-4-butoxyphenyl)-acetylen
1-(5-Hexylpyridin-2-yl)-2-(2,3-difluor-4-butoxyphenyl)-acetylen
1-(5-Heptylpyridin-2-yl)-2-(2,3-difluor-4-butoxyphenyl)-acetylen
1-(5-Methylpyridin-2-yl)-2-(2,3-difluor-4-pentyloxyphenyl)-acetylen
1-(5-Ethylpyridin-2-yl)-2-(2,3-difluor-4-pentyloxyphenyl)-acetylen
1-(5-Propylpyridin-2-yl)-2-(2,3-difluor-4-pentyloxyphenyl)-acetylen
1-(5-Butylpyridin-2-yl)-2-(2,3-difluor-4-pentyloxyphenyl)-acetylen
1-(5-Pentylpyridin-2-yl)-2-(2,3-difluor-4-pentyloxyphenyl)-acetylen
1-(5-Hexylpyridin-2-yl)-2-(2,3-difluor-4-pentyloxyphenyl)-acetylen
1-(5-Heptylpyridin-2-yl)-2-(2,3-difluor-4-pentyloxyphenyl)-acetylen

Beispiel A

Eine flüssigkristalline Phase, bestehend aus

| | |
|---|---|
| 9 % | r-1-Cyan-cis-4-(trans-4-propylcyclohexyl)-1-propyl-cyclohexan, |
| 5 % | r-1-Cyan-1-propyl-cis-4-(4'-propylbiphenyl-4-yl)-cyclohexan, |
| 26 % | 2-Fluor-4-ethyl-4'-[2-(trans-4-propyl-cyclohexyl)ethyl]-biphenyl, |
| 25 % | 2-Fluor-4-pentyl-4'-[2-(trans-4-propyl-cyclohexyl)ethyl]-biphenyl, |
| 23 % | 2-Fluor-4-ethyl-4'-[2-(trans-4-pentyl-cyclohexyl)ethyl]-biphenyl, |
| 5 % | 4-(trans-4-Propylcyclohexyl)-2'-fluor-4'-(trans-4-propylcyclohexyl)-biphenyl |
| und | |
| 7 % | 1-[4-(trans-4-Propylcyclohexyl)phenyl]-2-(5-methyl-pyridin-2-yl)-acetylen |

hat einen Klärpunkt von 109° und $\Delta\epsilon$ = -1,1.

**Patentansprüche**

**1.** Ethinderivate der Formel I

$$R^1\text{-}(A^1\text{-}Z^1)_m\text{-}A^3\text{-}C{\equiv}C\text{-}A^4\text{-}(Z^2\text{-}A^2)_n\text{-}R^2 \qquad I$$

worin

R¹ und R² → $R^1$ und $R^2$ jeweils unabhängig voneinander einen unsubstituierten, einen einfach durch -CN oder einen mindestens einfach durch Halogen substituierten Alkyl- oder Alkenylrest mit 1 bis 15 C-Atomen, wobei in diesen Resten auch eine oder mehrere $CH_2$-Gruppen jeweils unabhängig voneinander durch -O-, -S-, -CO-, -O-CO-, -O-COO-, -CO-O- oder -C≡C-so ersetzt sein können, daß Heteroatome nicht direkt miteinander verknüpft sind, einer der Reste $R^1$ und $R^2$ auch H, Halogen, -CN oder -NCS,

$A^1$ und $A^2$ jeweils unabhängig voneinander einen
a) 1,4-Phenylenrest,
worin auch eine oder mehrere CH-Gruppen durch N ersetzt sein können,
b) trans-1,4-Cyclohexylenrest,
worin auch eine oder zwei nicht benachbarte $CH_2$-Gruppen durch -O- und/oder -S- ersetzt sein können,
wobei die Reste a) und b) ein- oder mehrfach durch Halogen, Cyano und/oder $CH_3$ substituiert sein können,

$Z^1$ und $Z^2$ jeweils unabhängig voneinander -CO-O-, -O-CO-, $-CH_2O-$, $-OCH_2-$, $-CH_2CH_2-$, -C≡C- oder eine Einfachbindung,

m und n jeweils unabhängig voneinander 0 oder 1,
und

$A^3$ und $A^4$ jeweils unabhängig voneinander einen
a) 1,4-Phenylen-, Pyridin-2,5-diyl- oder Pyrimidin-2,5-diyl-Rest,
b) trans-1,4-Cyclohexylenrest, wobei die Reste a) und b) ein- oder mehrfach durch Halogen, Cyano und/oder $CH_3$ substituiert sein können,

bedeutet, mit den Maßgaben daß,

a) eine und nur eine der Gruppen $A^3$ oder $A^4$ einen Pyridin-, Pyrimidin- oder Cyclohexylenrest bedeutet,

b) $R^2$ Halogen, einen mindestens einfach durch Halogen substituierten Alkenylrest mit 1 bis 15 C-Atomen oder einen Perfluoralkylrest mit 1 bis 15 C-Atomen bedeutet, falls m = n = 0, $A^3$ trans-1,4-Cyclohexylen und $A^4$ unsubstituiertes 1,4-Phenylen bedeutet.

**2.** Ethinderderivate nach Anspruch 1, gekennzeichnet durch die Formel

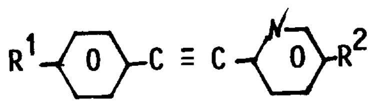

I af.

worin $R^1$ und $R^2$ die angegebene Bedeutung haben.

**3.** Ethinderderivate nach Anspruch 1, gekennzeichnet durch die Formel

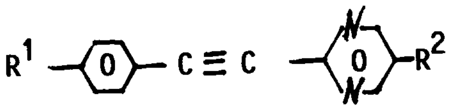

I ag

worin $R^1$ und $R^2$ die angegebene Bedeutung haben.

**4.** Ethinderderivate nach Anspruch 2, dadurch gekennzeichnet, daß $R^1$ Alkyl mit 2 bis 8 C-Atomen und $R^2$ Alkyl mit 1 bis 8 C-Atomen bedeutet.

**5.** Ethinderderivate nach Anspruch 2, dadurch gekennzeichnet, daß $R^1$ Alkyl mit 2 bis 8 C-Atomen und $R^2$ Alkyloxy mit 1 bis 8 C-Atomen bedeutet.

**6.** Ethinderderivate nach Anspruch 2, dadurch gekennzeichnet, daß $R^1$ Alkyloxy mit 2 bis 8 C-Atomen und $R^2$ Alkyl mit 1 bis 8 C-Atomen bedeutet.

**7.** Ethinderderivate nach Anspruch 3, dadurch gekennzeichnet, daß $R^1$ Alkyl mit 3 bis 6 C-Atomen und $R^2$ Alkyl mit 1 bis 8 C-Atomen bedeutet.

**8.** Ethinderderivate nach Anspruch 3, dadurch gekennzeichnet daß $R^1$ Alkyl mit 3 bis 6 C-Atomen und $R^2$ Alkyloxy mit 1 bis 8 C-Atomen bedeutet.

**9.** Ethinderderivate nach Anspruch 3, dadurch gekennzeichnet, daß $R^1$ Alkyloxy mit 3 bis 5 C-Atomen und $R^2$ Alkyl mit 1 bis 8 C-Atomen bedeutet.

**10.** Verwendung der Verbindungen der -Formel I nach Anspruch 1 als Komponenten flüssigkristalliner Phasen.

**11.** Flüssigkristalline Phase mit mindestens zwei flüssigkristallinen Komponenten, dadurch gekennzeichnet, daß sie mindestens eine Verbindung der Formel I enthält.

**12.** Flüssigkristalline Phase mit mindestens zwei flussigkristallinen Komponenten, dadurch gekennzeichnet, daß sie mindestens eine Verbindung enthaltend den strukturellen Bestandteil

$-A^3-C\equiv C-A^4-$

worin $A^3$ und $A^4$ jeweils unabhängig voneinander einen
a) 1,4-Phenylen-, Pyridin-2,5-diyl- oder Pyrimidin-2,5-diyl-Rest,
b) trans-1,4-Cyclohexylenrest,
bedeutet,

mit der Maßgabe, daß
eine und nur eine der Gruppen $A^3$ oder $A^4$ einen Pyridin- oder Pyrimidin-Rest bedeutet,

enthält.

**13.** Flüssigkristallanzeigeelement, dadurch gekennzeichnet, daß es eine flüssigkristalline Phase nach Anspruch 11 oder 12 enthält.

**Claims**

**1.** Ethyne derivatives of the formula I $R^1-(A^1-Z^1)_m-A^3-C\equiv C-A^4-(Z^{2-}A^2)_n-R^2$ I wherein

$R^1$ and $R^2$ in each case independently of one another are an alkyl or alkenyl radical having 1 to 15 C atoms which is unsubstituted, monosubstituted by -CN or at least monosubstituted by halogen, it also being possible for one or more $CH_2$ groups in these radicals in each case independently of one another to be replaced by -O-, -S-, -CO-, -O-CO-, -O-COO-, -CO-O- or -C≡C-such that heteroatoms are not linked directly to one another, and one of the radicals $R^1$ and $R^2$ can also be H, halogen, -CN or -NCS,

$A^1$ and $A^2$ in each case independently of one another are a
a) 1,4-phenylene radical, wherein one or more CH groups can also be replaced by N,
b) trans-1,4-cyclohexylene radical, wherein one or two non-adjacent $CH_2$ groups can also be replaced by -O- and/or -S-, it being possible for the radicals a) and b) to be mono- or polysubstituted by halogen, cyano and/or $CH_3$,

$Z^1$ and $Z^2$ in each case independently of one another are -CO-O-, -O-CO-, $-CH_2O-$, $-OCH_2-$, $-CH_2CH_2-$, -C≡C- or a single bond,

m and n in each case independently of one another are 0 or 1, and

$A^3$ and $A^4$ in each case independently of one another are a
a) 1,4-phenylene, pyridine-2,5-diyl or pyrimidine-2,5-diyl radical,
b) trans-1,4-cyclohexylene radical, it being possible for the radicals a) and b) to be substituted once or more than once by halogen, cyano and/or $CH_3$,
with the provisos that

a) one and only one of the groups $A^3$ or $A^4$ is a pyridine, pyrimidine or cyclohexylene radical,
b) $R^2$ is halogen, an alkenyl radical having 1 to 15 C atoms which is at least monosubstituted by halogen or a perfluoroalkyl radical having 1 to 15 C atoms, if m = n = 0, $A^3$ is trans-1,4-cyclohexylene and $A^4$ is unsubstituted 1,4-phenylene.

**2.** Ethyne derivatives according to Claim 1, characterised by the formula

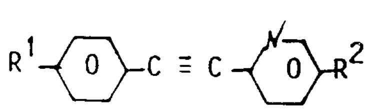

Iaf

wherein $R^1$ and $R^2$ have the meaning given.

**3.** Ethyne derivatives according to Claim 1, characterised by the formula

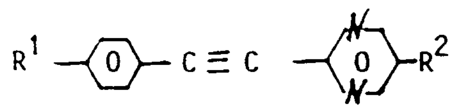

Iag

wherein $R^1$ and $R^2$ have the meaning given.

**4.** Ethyne derivatives according to Claim 2, characterised in that
$R^1$ is alkyl having 2 to 8 C atoms and
$R^2$ is alkyl having 1 to 8 C atoms.

**5.** Ethyne derivatives according to Claim 2, characterised in that
$R^1$ is alkyl having 2 to 8 C atoms and
$R^2$ is alkyloxy having 1 to 8 C atoms.

**6.** Ethyne derivatives according to Claim 2, characterised in that
$R^1$ is alkyloxy having 2 to 8 C atoms and
$R^2$ is alkyl having 1 to 8 C atoms.

**7.** Ethyne derivatives according to Claim 3, characterised in that
$R^1$ is alkyl having 3 to 6 C atoms and
$R^2$ is alkyl having 1 to 8 C atoms.

**8.** Ethyne derivatives according to Claim 3, characterised in that
$R^1$ is alkyl having 3 to 6 C atoms and
$R^2$ is alkyloxy having 1 to 8 C atoms.

**9.** Ethyne derivatives according to Claim 3, characterised in that
$R^1$ is alkyloxy having 3 to 5 C atoms and
$R^2$ is alkyl having 1 to 8 C atoms.

**10.** Use of the compounds of the formula I according to Claim 1 as components of liquid crystal phases.

**11.** Liquid crystal phase with at least two liquid crystal components, characterised in that it contains at least one compound of the formula I.

**12.** Liquid crystal phase with at least two liquid crystal components, characterized in that it contains at least one compound containing the structural constituent

$-A^3-C\equiv C-A^4-$

wherein $A^3$ and $A^4$ in each case independently of one another are a
a) 1,4-phenylene, pyridine-2,5-diyl or pyrimidine-2,5-diyl radical,
b) trans-1,4-cyclohexylene radical, with the proviso that one and only one of the groups $A^3$ or $A^4$ is a pyridine or pyrimidine radical.

**13.** Liquid crystal display element, characterised in that it contains a liquid crystal phase according to Claim 11 or 12.

## Revendications

**1.** Dérivés de l'éthyne de formule I

$R^1-(A^1-Z^1)_m-A^3-C\equiv C-A^4-(Z^2-A^2)_n-R^2$ I

dans laquelle

$R^1$ et $R^2$ représentent chacun, indépendamment l'un de l'autre, un groupe alkyle ou alcényle en C 1-C 15 non substitué, substitué par un groupe -CN ou substitué par au moins un halogène, et dans lesquels un ou plusieurs groupes $CH_2$ peuvent être remplacés chacun, indépendamment les uns des autres, par -O-, -S-, -CO-, -O-CO-, -O-COO-, -CO-O- ou -C≡C-, à condition que les hétéroatomes ne soient pas reliés directement entre eux, l'un des symboles $R^1$ et $R^2$ pouvant également représenter H, un halogène, un groupe -CN ou -NCS,

$A^1$ et $A^2$ représentent chacun, indépendamment l'un de l'autre,

a) un groupe 1,4-phénylène dans lequel un ou plusieurs groupes CH peuvent être remplacés par N,

b) un groupe trans-1,4-cyclohexylène dans lequel un ou deux groupes $CH_2$ non voisins peuvent être remplacés par -O- et/ou -S-, les groupes a) et b) pouvant porter un ou plusieurs substituants choisis parmi les halogènes, les groupes cyano et/ou $CH_3$,

$Z^1$ et $Z^2$ représentent chacun, indépendamment l'un de l'autre, -CO-O-, -O-CO-, $-CH_2O-$, $-OCH_2-$, $-CH_2CH_2-$, -C≡C- ou une liaison simple,

m et n sont égaux chacun, indépendamment l'un de l'autre, à 0 ou 1, et

$A^3$ et $A^4$ représentent chacun, indépendamment l'un de l'autre,

a) un groupe 1,4-phénylène, pyridine-2,5-diyle ou pyrimidine-2,5-diyle,

b) un groupe trans-1,4-cyclohexylène, les groupes a) et b) pouvant porter un ou plusieurs substituants choisis parmi les halogènes, les groupes cyano et/ou $CH_3$.

sous réserve que

a) un et un seul des symboles $A^3$ et $A^4$ représente un groupe pyridinique, pyrimidinique ou cyclohexylène,

b) $R^2$ représente un halogène, un groupe alcényle en C 1-C 15 substitué au moins une fois par un halogène ou un groupe perfluoralkyle en C 1-C 15 lorsque $m = n = 0$, $A^3$ représente un groupe trans-1,4-cyclohexylène et $A^4$ un groupe 1,4-phénylène non substitué.

**2.** Dérivés de l'éthyne selon revendication 1, caractérisés par la formule

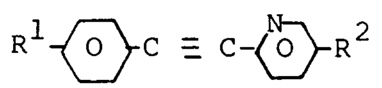

I af

dans laquelle $R^1$ et $R^2$ ont les significations indiquées ci-dessus.

**3.** Dérivés de l'éthyne selon revendication 1, caractérisés par la formule

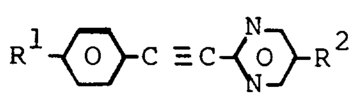

I ag

dans laquelle $R^1$ et $R^2$ ont les significations indiquées ci-dessus.

**4.** Dérivés de l'éthyne selon revendication 2, caractérisés en ce que $R^1$ représente un groupe alkyle en C 2-C 8, et $R^2$ représente un groupe alkyle en C 1-C 8.

**5.** Dérivés de l'éthyne selon revendication 2, caractérisés en ce que $R^1$ représente un groupe alkyle en C 2-C 8 et $R^2$ un groupe alkyloxy en C 1-C 8.

**6.** Dérivés de l'éthyne selon revendication 2, caractérisés en ce que $R^1$ représente un groupe alkyloxy en C 2-C 8 et $R^2$ un groupe alkyle en C 1-C 8.

**7.** Dérivés de l'éthyne selon revendication 3, caractérisés en ce que $R^1$ représente un groupe alkyle en C 3-C 6 et $R^2$ un groupe alkyle en C 1-C 8.

**8.** Dérivés de l'éthyne selon revendication 3, caractérisés en ce que $R^1$ représente un groupe alkyle en C 3-C 6 et $R^2$ un groupe alkyloxy en C 1-C 8.

**9.** Dérivés de l'éthyne selon revendication 3, caractérisés en ce que $R^1$ représente un groupe alkyloxy en C 3-C 5 et $R^2$ un groupe alkyle en C 1-C 8.

**10.** Utilisation des composés de formule I de la revendication 1 en tant que composants de phases à cristaux liquides.

**11.** Phase à cristaux liquides, à au moins deux composants à cristaux liquides, caractérisée en ce qu'elle contient au moins un composé de formule I.

**12.** Phase à cristaux liquides, à au moins deux composants à cristaux liquides, caractérisée en ce qu'elle contient au moins un composé contenant lui-même un motif de structure

$-A^3-C\equiv C-A^4-$

dans laquelle $A^3$ et $A^4$ représentent chacun, indépendamment l'un de l'autre,

a) un groupe 1,4-phénylène, pyridine-2,5-diyle ou pyrimidine-2,5-diyle,

b) un groupe trans-1,4-cyclohexylène,

sous réserve qu'un et un seul des groupes $A^3$ et $A^4$ est un groupe pyridinique ou pyrimidinique.

**13.** Elément d'affichage à cristaux liquides, caractérisé en ce qu'il contient une phase à cristaux liquides selon revendication 11 ou 12.